# EUROPEAN PATENT APPLICATION

(11) **EP 3 789 384 A1**
(43) Date of publication of application: **10.03.2021**
(21) Application number: 19791533.3
(22) Date of filing: 23.04.2019
(51) Int. Cl.: C07D 401/04, C07D 403/04, C07D 411/04, C07D 403/14, A61K 31/4196, A61K 31/4439, A61P 3/00, A61P 9/00, A61P 25/00, A61P 29/00, A61P 35/00, A61P 37/00

(54) **FORMAMIDE COMPOUND, PREPARATION METHOD THEREFOR AND APPLICATION THEREOF**

(30) Priority: 28.04.2018 CN 201810404758
(71) Applicant: Shenzhen Chipscreen Biosciences Co., Ltd., Shenzhen, Guangdong 518057 (CN)
(72) Inventor: YANG, Qianjiao, Shenzhen, Guangdong 518057 (CN); LU, Xianping, Shenzhen, Guangdong 518057 (CN); LI, Zhibin, Shenzhen, Guangdong 518057 (CN); PAN, Desi, Shenzhen, Guangdong 518057 (CN); SHAN, Song, Shenzhen, Guangdong 518057 (CN); WANG, Xiaoliang, Shenzhen, Guangdong 518057 (CN); SONG, Yonglian, Shenzhen, Guangdong 518057 (CN); ZHANG, Kun, Shenzhen, Guangdong 518057 (CN)
(74) Representative: Lavoix
(86) International application number: PCT/CN2019/083829
(87) International publication number: WO 2019/206120

(57) **Abstract**

The present invention relates to a formamide compound, a preparation method therefor and an application thereof. The structure of the compound is shown in formula (I), and the definition of each variable in the formula is as provided in the description. The compound is capable of inhibiting the activity of ASK1 kinase. The compound of the present invention may be used in the treatment/prevention of diseases associated with ASK1 kinase, such as inflammatory diseases, metabolic diseases, autoimmune diseases, cardiovascular diseases, neurodegenerative diseases, cancers and other diseases.

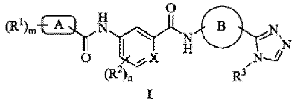

## Description

The present application claims priority to Chinese Patent Application No. 201810404758.X entitled "FORMAMIDE COMPOUND, PREPARATION METHOD THEREFOR AND APPLICATION THEREOF" filed with State Intellectual Property Office on April 28, 2018, which is incorporated herein by reference in its entirety.

### FIELD

The present invention belongs to the field of medical technology, and relates to a formamide compound capable of inhibiting the activity of ASK1 kinase, a preparation method therefor, and a pharmaceutical composition comprising the compound as an active ingredient and a pharmaceutical application thereof. The compound of the present invention can function as an inhibitor targeting ASK1 kinase for the treatment/prevention of diseases associated with this target, such as inflammatory diseases, metabolic diseases, autoimmune diseases, cardiovascular diseases, neurodegenerative diseases, cancers and other diseases.

### BACKGROUND

Mitogen-activated protein kinases (MAPKs) are Ser/Thr protein kinases widely distributed in the cytoplasm, which are important transmitters transducing signals from the cell surface to the nucleus. The MAPKs signaling pathway consists of a three-stage kinase model, comprising MEK kinase (MAP3K), MAPK kinase (MAP2K), and MAP kinase (MAPK). This pathway can initiate the three-stage kinase cascade from MAP3K to MAP2K and then to MAPK in response to a variety of different extracellular stimuli, such as cytokines, cellular stress, neurotransmitter, and the like, and activate different MAPKs signaling pathways by acting on different reaction substrates, thereby regulating a variety of different pathological and physiological processes such as gene expression, cell growth, differentiation, apoptosis, metabolism, and participating in inflammatory responses (Cargnello M., Roux P. P., 2011, Microbiol. Mol. Biol. Rev., 75: 50-83).

Apoptosis signal-regulating kinase 1 (ASK1) is one of the MAP3K family members. ASK1 can be first activated by a variety of different stimuli such as oxidative stress, reactive oxygen species (ROS), lipopolysaccharide (LPS), tumor necrosis factor (TNF-α), endoplasmic reticulum (ER) stress, osmotic pressure, inflammation and the like, and then MAP2K is activated and phosphorylated to activate MAPK, such as c-Jun N-terminal protein kinase (JNK) and p38 MAPK. It can be seen that ASK1 plays a key role in a variety of cell biological processes, including apoptosis, differentiation, and inflammation (Soga M., Matsuzawa A., Ichijo H., 2012, Int. J. Cell Biol., 2012: 1-5).

Reports suggest that the activation of ASK1 plays an important role in a variety of diseases, such as inflammatory diseases, metabolic diseases, autoimmune diseases, cardiovascular diseases, neurodegenerative diseases, cancers and other diseases (Soga M., Matsuzawa A., Ichijo H., 2012, Int. J. Cell Biol., 2012: 1-5; Hayakawa R., Hayakawa T., Takeda K., et al, 2012,Proc.Jpn.Acad.Ser.BPhys.Biol.Sci., 88: 434-453). Therefore, discovery of pharmaceutically active molecules capable of inhibiting the activity of ASK1 will bring significant benefits to patients with the aforementioned diseases.

So far, the published patent applications involving ASK1 inhibitors include WO2009027283 involving triazolopyridines, WO2011041293 involving pyrazolo[1,5-A]pyrimidines, WO2011008709 involving aromatic ring/aromatic heterocyclic amines, US20120004267 involving heterocyclic amines and US20170173031 involving thiazolamines. As mentioned above, the activation of ASK1 is associated with a variety of diseases. Inhibitors of ASK1, as drugs, have important clinical value and good application prospects in the medical field. However, there is currently no drug approved for marketing in the world. Therefore, we expect to develop new ASK1 inhibitors to meet the unmet clinical needs.

The present invention provides a novel cycloalkylformamide ASK1 inhibitor for the treatment/prevention of diseases associated with this target, such as inflammatory diseases, metabolic diseases, autoimmune diseases, cardiovascular diseases, neurodegenerative diseases, cancers and other diseases. At the same time, these compounds or pharmaceutical compositions comprising them as active ingredients and the like can maximize the clinical efficacy of these diseases within safe treatment window.

### SUMMARY

One aspect of the present invention relates to a cycloalkylformamide compound shown in the following formula I that can inhibit the activity of ASK1 kinase, including a derivative thereof such as a pharmaceutically acceptable salt, a hydrate, other solvates, a stereoisomer and a prodrug thereof.

Another aspect of the present invention relates to a method for preparing the compounds described herein.

Another aspect of the present invention relates to a pharmaceutical composition comprising the compound of the present invention as an active ingredient, and the clinical application of the compound or pharmaceutical composition of the present invention for the treatment/prevention of a disease associated with ASK1 kinase, and the use of the compound or pharmaceutical combination of the present invention in the manufacture of a medicament for the treatment and/or prevention of a disease associated with ASK1 kinase. Correspondingly, the present invention also relates to a method for treating and/or preventing disease associated with ASK1 kinase comprising administering the compound or pharmaceutical composition of the present invention to a subject in need thereof.

The present invention relates to a compound of formula I, 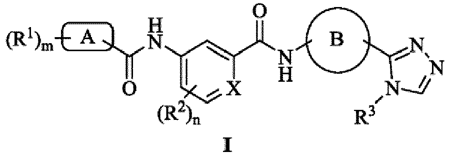 wherein,
R¹ is one or more same or different substituents independently selected from H, halogen, CN, C₁-C₄ alkyl, C₁-C₄ haloalkyl, NH₂, COOH, C₁-C₄ alkylamino, C₁-C₄ alkyloxy and Ar¹;
   wherein, Ar¹ is selected from a benzene ring and a pyridine ring, wherein the benzene ring and the pyridine ring may be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
R² is one or more same or different substituents independently selected from H, halogen, CN, C₁-C₄ alkyl and C₁-C₄ haloalkyl;
R³ is C₁-C₄ alkyl, C₃-C₆ cycloalkyl, C₁-C₄ haloalkyl, halo C₃-C₆ cycloalkyl, cyano substituted C₁-C₄ alkyl, C₃-C₆ heterocycloalkyl, hydroxy substituted C₁-C₄ alkyl or C₁-C₄ alkoxy substituted C₁-C₄ alkyl;
X is selected from C and N;
A is selected from C₃-C₇ cycloalkyl and C₃-C₇ heterocycloalkyl;
B is an aromatic ring,
   preferably selected from a benzene ring, a pyridine ring, a thiazole ring, a furan ring, a thiophene ring, a pyrrole ring, a pyrazole ring, a oxazole ring, a isoxazole ring and a quinoline ring, and the aromatic ring may be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
m is an integer from 1 to 5; and
n is an integer from 1 to 4;
or a prodrug, a stereoisomer, a pharmaceutically acceptable salt, a hydrate or other solvates thereof.

In a preferred aspect, the present invention relates to a compound of formula I, wherein,
R¹ is one or more same or different substituents independently selected from H, halogen, CN, C₁-C₄ alkyl, C₁-C₄ haloalkyl, NH₂, COOH, C₁-C₄ alkylamino, C₁-C₄ alkyloxy and Ar¹;
   wherein, Ar¹ is selected from a benzene ring and a pyridine ring, wherein the benzene ring and the pyridine ring may be substituted by one or more same or different substituents independently selected from H, halogen, CN, C₁-C₄ Alkyl, C₁-C₄ haloalkyl, NH₂, C₁-C₄ alkylamino and C₁-C₄ alkyloxy;
R² is one or more same or different substituents independently selected from H, halogen, CN and C₁-C₄ alkyl;
R³ is C₁-C₄ alkyl, C₃-C₆ cycloalkyl, C₁-C₄ haloalkyl, halo C₃-C₆ cycloalkyl, cyano substituted C₁-C₄ alkyl, C₃-C₆ heterocycloalkyl, hydroxy substituted C₁-C₄ alkyl and C₁-C₄ alkoxy substituted C₁-C₄ alkyl;
X is selected from C and N;
A is selected from C₃-C₇ cycloalkyl and C₃-C₇ heterocycloalkyl;
B is an aromatic ring, preferably selected from a benzene ring, a pyridine ring, a thiazole ring, a furan ring, a thiophene ring, a pyrrole ring, a pyrazole ring, a oxazole ring, a isoxazole ring and a quinoline ring, and the aromatic ring may be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
m is an integer from 1 to 5; and
n is an integer from 1 to 4;
or a prodrug, a stereoisomer, a pharmaceutically acceptable salt, a hydrate or other solvates thereof.

In a more preferred aspect, the present invention relates to a compound of formula I, wherein:
R¹ is one or more same or different substituents independently selected from H, halogen, CN, C₁-C₄ alkyl, C₁-C₄ haloalkyl, NH₂, COOH, C₁-C₄ alkylamino and C₁-C₄ alkyloxy;
R² is one or more same or different substituents independently selected from H, halogen, CN and C₁-C₄ alkyl;
R³ is C₁-C₄ alkyl, C₃-C₆ cycloalkyl, C₁-C₄ haloalkyl, halo C₃-C₆ cycloalkyl, cyano substituted C₁-C₄ alkyl, C₃-C₆ heterocycloalkyl, hydroxy substituted C₁-C₄ alkyl and C₁-C₄ alkoxy substituted C₁-C₄ alkyl;
X is selected from C and N;
A is selected from C₃-C₇ cycloalkyl and C₃-C₇ heterocycloalkyl;
B is an aromatic ring, preferably selected from a benzene ring, a pyridine ring, a thiazole ring, a furan ring, a thiophene ring, a pyrrole ring, a pyrazole ring, a oxazole ring, a isoxazole ring and a quinoline ring, and the aromatic ring may be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
m is an integer from 1 to 5; and
n is an integer from 1 to 4;
or a prodrug, a stereoisomer, a pharmaceutically acceptable salt, a hydrate or other solvates thereof.

In another more preferred aspect, the present invention relates to a compound of formula I, wherein:
R¹ is one or more same or different substituents independently selected from H, halogen, CN, C₁-C₄ alkyl, C₁-C₄ haloalkyl, NH₂, COOH, C₁-C₄ alkylamino and C₁-C₄ alkyloxy;
R² is one or more same or different substituents independently selected from H, halogen, CN and C₁-C₄ alkyl;
R³ is C₁-C₄ alkyl, C₃-C₆ cycloalkyl, C₁-C₄ haloalkyl, halo C₃-C₆ cycloalkyl and cyano substituted C₁-C₄ alkyl;
X is selected from C and N;
A is selected from C₃-C₇ cycloalkyl and C₃-C₇ heterocycloalkyl;
B is an aromatic ring, preferably selected from a benzene ring, a pyridine ring, a thiazole ring, a furan ring, a thiophene ring, a pyrrole ring, a pyrazole ring, a oxazole ring, a isoxazole ring and a quinoline ring, and the aromatic ring may be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
m is an integer from 1 to 5; and
n is an integer from 1 to 4;
or a prodrug, a stereoisomer, a pharmaceutically acceptable salt, a hydrate or other solvates thereof.

In another yet more preferred aspect, the present invention relates to a compound of formula I, wherein:
R¹ is one or more same or different substituents independently selected from H, halogen, CN, C₁-C₄ alkyl, C₁-C₄ haloalkyl, NH₂, COOH, C₁-C₄ alkylamino and C₁-C₄ alkyloxy;
R² is one or more same or different substituents independently selected from H, halogen, CN and C₁-C₄ alkyl;
R³ is C₁-C₄ alkyl, C₃-C₆ cycloalkyl, C₁-C₄ haloalkyl, halo C₃-C₆ cycloalkyl and cyano substituted C₁-C₄ alkyl;
X is selected from C and N;
A is selected from C₃-C₅ cycloalkyl and C₃-C₅ heterocycloalkyl;
B is an aromatic ring, preferably selected from a benzene ring, a pyridine ring and a thiazole ring, and the aromatic ring may be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
m is an integer from 1 to 5; and
n is an integer from 1 to 4;
or a prodrug, a stereoisomer, a pharmaceutically acceptable salt, a hydrate or other solvates thereof.

In another yet more preferred aspect, the present invention relates to a compound of formula I, wherein:
R¹ is one or more same or different substituents independently selected from H, halogen, CN and C₁-C₄ alkyl;
R² is one or more same or different substituents independently selected from H, halogen, CN and methyl;
R³ is C₁-C₄ alkyl, C₃-C₆ cycloalkyl, C₁-C₄ haloalkyl, halo C₃-C₆ cycloalkyl or cyano substituted C₁-C₄ alkyl;
X is selected from C and N;
A is selected from C₃-C₄ cycloalkyl and C₃-C₄ heterocycloalkyl;
B is an aromatic ring, preferably selected from a benzene ring, a pyridine ring and a thiazole ring, and the aromatic ring may be substituted by one or more same or different substituents independently selected from H, halogen, CN, C₁-C₄ alkyl and C₁-C₄ haloalkyl;
m is an integer from 1 to 5; and
n is an integer from 1 to 4;
or a prodrug, a stereoisomer, a pharmaceutically acceptable salt, a hydrate or other solvates thereof.

In another yet more preferred aspect, the present invention relates to a compound of formula I, wherein:
R¹ is one or more same or different substituents independently selected from H, halogen and CN;
R² is one or more same or different substituents independently selected from H, F, Cl, CN and methyl;
R³ is C₁-C₄ alkyl, C₃-C₆ cycloalkyl, C₁-C₄ haloalkyl, halo C₃-C₆ cycloalkyl or cyano substituted C₁-C₄ alkyl;
X is selected from C and N;
A is selected from C₃-C₄ cycloalkyl and C₃-C₄ heterocycloalkyl;
B is an aromatic ring, preferably selected from a benzene ring, a pyridine ring and a thiazole ring, and the aromatic ring may be substituted by one or more same or different substituents independently selected from H, halogen, CN, methyl and CF₃;
m is an integer from 1 to 5; and
n is an integer from 1 to 4;
or a prodrug, a stereoisomer, a pharmaceutically acceptable salt, a hydrate or other solvates thereof.

In a more preferred aspect, the present invention relates to a compound of formula I, wherein:
R¹ is H;
R² is one or more same or different substituents independently selected from H, F, Cl, CN and methyl;
R³ is C₁-C₄ alkyl, C₃-C₆ cycloalkyl, C₁-C₄ haloalkyl, halo C₃-C₆ cycloalkyl or cyano substituted C₁-C₄ alkyl;
X is selected from C and N;
A is selected from C₃-C₄ cycloalkyl;
B is an aromatic ring, preferably selected from a benzene ring, a pyridine ring and a thiazole ring, and the aromatic ring may be substituted by one or more same or different substituents independently selected from H, halogen, CN, methyl and CF₃;
m is 1; and
n is an integer from 1 to 3;
or a prodrug, a stereoisomer, a pharmaceutically acceptable salt, a hydrate or other solvates thereof.

In a particularly more preferred aspect, the present invention relates to a compound of formula I, wherein:
R¹ is H;
R² is one or more same or different substituents independently selected from H, F, Cl, CN and methyl;
R³ is C₁-C₄ alkyl, C₃-C₆ cycloalkyl, C₁-C₄ haloalkyl, halo C₃-C₆ cycloalkyl or cyano substituted C₁-C₄ alkyl;
X is selected from C and N;
A is selected from C₃-C₄ cycloalkyl;
B is an aromatic ring, preferably selected from a benzene ring, a pyridine ring and a thiazole ring, and the aromatic ring may be substituted by one or more same or different substituents independently selected from H, halogen, CN, methyl and CF₃;
m is 1; and
n is an integer from 1 to 2;
or a prodrug, a stereoisomer, a pharmaceutically acceptable salt, a hydrate or other solvates thereof.

### DETAILED DESCRIPTION

The "halogen" as described in the present invention is fluorine, chlorine, bromine or iodine, preferably fluorine or chlorine.

The "alkyl" as described in the present invention includes straight or branched chain alkyl. The C₁-C₄ alkyl as described in the present invention refers to an alkyl having 1 to 4 carbon atoms, preferably methyl, ethyl, propyl or isopropyl, n-butyl, isobutyl or tert-butyl. The alkyl in the compound of the present invention may be optionally substituted or unsubstituted, and the substituent may include alkyl, halogen, alkoxy, haloalkyl, cyano, and hydroxy. Examples of the alkyl of the present invention include methyl, ethyl, n-propyl, isopropyl, n-butyl, isobutyl, and tert-butyl.

The "cycloalkyl" as described in the present invention includes 3-7 membered cycloalkyl, preferably cyclopropyl, cyclobutyl, cyclopentyl, and cyclohexyl. The cycloalkyl in the compound of the present invention may be optionally substituted or unsubstituted, and the substituent may include alkyl, halogen, alkoxy, hydrocarbyl, and hydroxyl.

The "heterocycloalkyl" as described in the present invention includes 3-7 membered heterocycloalkyl. The heterocycloalkyl in the compound of the present invention may be optionally substituted or unsubstituted, and the substituent may include alkyl, halogen, alkoxy, haloalkyl, cyano, and hydroxyl.

The "alkoxy" as described in the present invention refers to a group formed by connecting the above alkyl and oxygen atom, wherein the oxygen atom has the ability to bond freely, such as methoxy, ethoxy, propoxy, butoxy, isopropoxy, tert-butoxy, cyclopropoxy, and the like.

The "alkylamino" as described in the present invention refers to a group formed by connecting the above alkyl and amino, such as methylamino, ethylamino, 4-dimethylamino and the like.

As used herein, "substituted by one or more substituents" as referred herein means substituted by one or more than one substituents, for example, 1, 2, 3 or 4 substituents; preferably, 1, 2 or 3 substituents.

As used herein, "other solvates" means a solvate formed with a solvent other than water.

"Pharmaceutically acceptable" as described in the present invention is understood to be suitable for human and animal use within a reasonable medical scope, tolerable and without unacceptable side effects including toxicity, allergic reaction, irritation and complication.

The present invention relates to a pharmaceutical composition comprising the above compound of formula I or a prodrug, a stereoisomer, a pharmaceutically acceptable salt, a hydrate or other solvates thereof as an active ingredient.

The compound of the present invention can optionally be used in combination with one or more other active ingredients, and the respective dosages and ratios of which can be adjusted by those skilled in the art according to specific diseases, specific conditions of patients, clinical needs and the like.

The examples and preparation examples provided in the present invention further illuminate and illustrate the compound of the present invention and the preparation method therefor. It should be understood that the following preparation examples and examples do not limit the scope of the present invention in any way.

The following synthesis route describes the preparation method for the compound of formula I of the present invention. The raw materials, reagents, catalysts, solvents, and the like used in the following synthesis scheme can be prepared by methods well known to those of ordinary skill in the organic chemistry field or are commercially available. All final derivatives of the present invention can be prepared by the methods described in the schematic diagram or similar methods, which are well known to those of ordinary skill in the organic chemistry field. All variables used in these schemes are defined below or in the claims.

**Preparation method:** The definitions of the following variables are as described above, and the definition of new variables is as described in this section. In addition, the compound of formula I and the related intermediates can be purified by common separation methods, such as extraction, recrystallization, and silica gel column chromatography. The 200-300 mesh silica gel and thin-layer chromatography silica gel plates used were all produced by Qingdao Ocean Chemical Factory. The chemical reagents used were analytically pure or chemically pure commercially available products of general reagents, and are used without further purification.
(a) the key intermediate **II** can be prepared by the following exemplary synthesis method:
   the commercially available **II-1** is methylated or ethylated by common methods such as acyl chloride/methanol (CH₃OH) or ethanol (C₂H₅OH), and sulfuric acid/CH₃OH or C₂H₅OH to obtain **II**-**2**. Under the action of common reducing agents (including but not limited to iron powder/ammonium chloride (Fe/NH₄Cl) or iron powder/hydrochloric acid, etc.), **II-2** is dissolved in a mixed solvent of CH₃OH or C₂H₅OH and water, and reacted at 70-100 °C for about 2-4 h to obtain **II-3**. **II-3** (homemade or commercially available) is dissolved in common solvents (including but not limited to dichloromethane (CH₂Cl₂), tetrahydrofuran (THF), *N,N*'-dimethylformamide (DMF) or pyridine (Py), etc.), acyl chloride **II-4** is added dropwise to the aforementioned solution under the catalysis of common bases (such as triethylamine (TEA) and *N,N*'-diisopropylethylamine (DIPEA), etc.), or carboxylic acid **II-4** is added dropwise to the aforementioned solution under the action of a common condensing agent to obtain **II-5.** At room temperature, **II-5** is dissolved in a mixed solvent of CH₃OH, C₂H₅OH or THF and water, and is subjected to the carboxylic ester hydrolysis with inorganic bases such as lithium hydroxide (LiOH), sodium hydroxide (NaOH) and the like, and the key intermediate **II** is usually obtained after the reaction is completed overnight. The common condensing agent described in this route is for example, but not limited to, O-(7-azabenzotriazol-1-yl)-*N,N,N',N*'-tetramethylurea hexafluorophosphate (HATU), 1-hydroxybenzotriazole (HOBt), 1*H*-benzotriazol-1-yloxytripyrrolidinyl hexafluorophosphate (PyBOP) and 1-propyl phosphoric anhydride (T₃P). 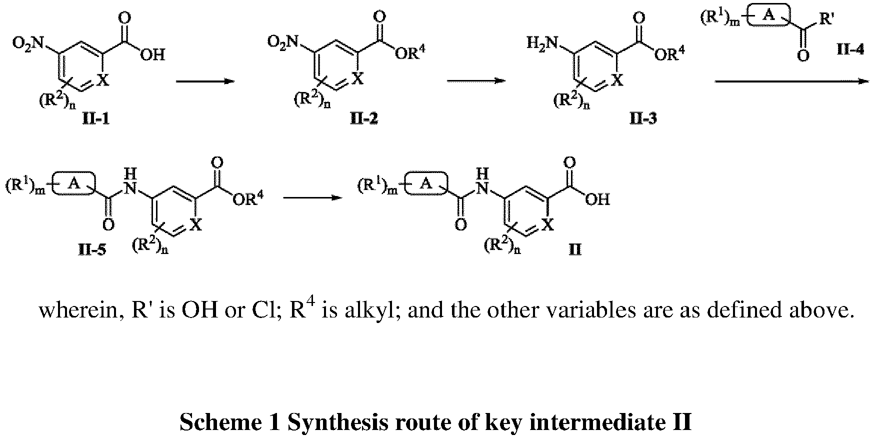
(b) By referring to a reference document US20110009410 and through research, it is found that the present invention can obtain intermediate **III** without using any protecting group on the amino group. Compared with the method reported in the document US20110009410, the method of the present invention shortens the reaction steps, saves time, saves costs, and the total yield is increased from about 25% reported in the document to about 30%-66% for most compounds in the method. The key intermediate **III** can be prepared by the following exemplary synthesis method:
   the commercially available **III-1** is reacted with hydrazine hydrate in a suitable protic solvent for about 1-3 h under reflux to obtain **III-2;** then **III-2** is reacted with *N,N*'-dimethyl formamide dimethyl acetal (DMF-DMA) for about 3-10 h under reflux to obtain **III-3;** and then **III-3** is reacted with the commercially available amine **III-4** in acetonitrile/glacial acetic acid (CH₃CN/AcOH) for at least 24 h under reflux to obtain the key intermediate **III.** The protic solvent described in this route can be but not limited to CH₃OH, C₂H₅OH and the like. 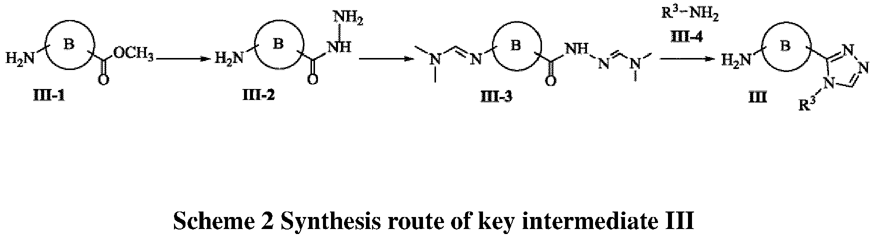
(c) The key intermediate **III** can be prepared by the following exemplary other synthesis methods:
   Under N₂ protection, the commercially available **III'-1** is dissolved in THF or 1,4-dioxane, and reacted with n-butyllithium (n-BuLi) and CO₂ for about 1-3 h at a low temperature of -70 °C to convert the halogen into a carboxyl so as to obtain **III'-2.** Then, **III'-2** and the commercially available amine **III-4** are dissolved in a solvent such as CH₂Cl₂, THF or DMF, and reacted under the catalysis of a common condensing agent and a common base for about 3-5 h at room temperature to obtain **III'-3.** The oxo **III'-3** is converted to the thio **III'-4** under the action of Lawesson's Reagent and the reaction temperature from room temperature to 120 °C overnight. **III'-4** is reacted with hydrazine hydrate for about 1-3 h under reflux to obtain **III'-5.** At room temperature, **III'-5** is dissolved in C₂H₅OH, triethyl orthoformate (CH(OC₂H₅)₃) is added, and the ring-closure reaction is achieved by sulfuric acid catalysis, and **III'-6** is obtained after about 1-5 h. Under N₂ protection, **III'-6** is dissolved in a mixed solvent of 1,4-dioxane and water, benzophenone imine is added, and the C-N coupling reaction is completed under the catalysis of a palladium reagent, a common ligand and a base, and then **III'-7** is obtained after the reaction is left overnight under reflux. **III'-7** is hydrolyzed by dilute hydrochloric acid for about 24 h at room temperature to obtain the key intermediate **III.** The common condensing agent described in this route is for example, but not limited to, HATU, HOBt, PyBOP, T₃P and the like; the base is but not limited to TEA, DIPEA, potassium carbonate (K₂CO₃), cesium carbonate (Cs₂CO₃), sodium tert-butoxide (t-BuONa) and the like; the palladium reagent is but not limited to Tris(dibenzylideneacetone)dipalladium (Pd₂(dba)₃) and the dichloromethane complex thereof, palladium acetate (Pd(OAc)₂) and the like; and the ligand is but not limited to 4,5-bisdiphenylphosphine-9,9-dimethylxanthene (Xantphos), 2-biscyclohexylphosphine-2',6'-dimethoxybiphenyl (Sphos), 1,1'-binaphthyl-2,2'-bisdiphenylphosphine (BINAP) and the like. 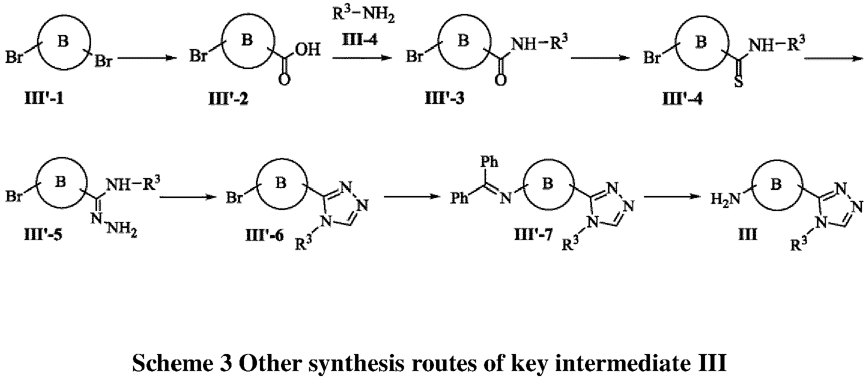
(d) The compound of formula **I** of the present invention can be prepared by the following exemplary synthesis method:
   The key intermediate **II** is prepared into acyl chloride through thionyl chloride (SOCl₂), oxalyl chloride ((COCl)₂), phosphorus trichloride (PCl₃) or phosphorus pentachloride (PCl₅), and then the active intermediate and the key intermediate **III** are dissolved in an ultra-dry solvent, such as CH₂Cl₂, THF, DMF or Py, etc., and a common basic catalyst is added to obtain the compound of formula **I.** In addition, the compound of formula **I** can also be obtained by using a common condensing agent for example, but not limited to, HATU, HOBt, PyBOP, T₃P and the like. The basic catalyst described in this route is for example, but not limited to, TEA, DIPEA K₂CO₃, and the like. 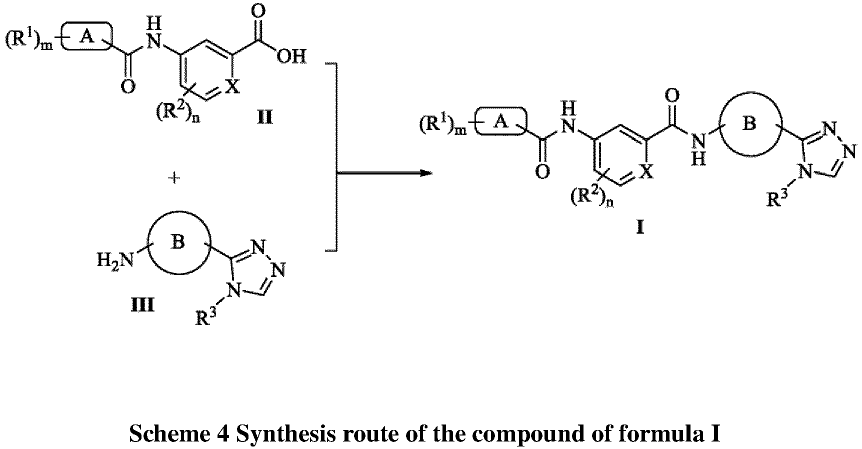
(e) The structural formula **I** of the present invention can also be obtained from the starting materials **II** and **IV-1** using a similar synthesis method to **Scheme 2,** as shown in Scheme 5 below. In addition, formula **I** can also be obtained by a condensation reaction between the compound of formula **V** and the compound of formula **II-4** under the catalysis of a base. 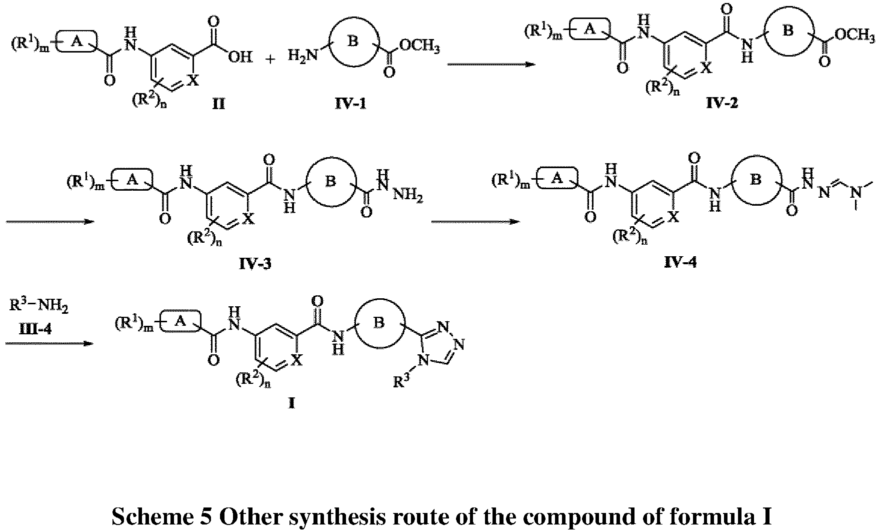 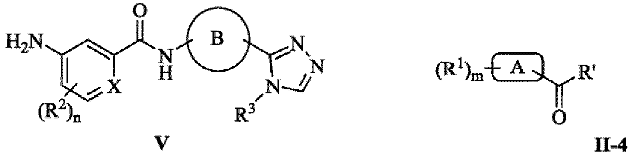 Wherein, R' is OH or Cl; R⁴ is alkyl; and the other variables are as defined above.

### LC-MS analysis method:

Mass spectrometry conditions: instrument, Thermo MSQ Plus; ion source, ESI (EA+ EA-); cone voltage, 30 V; capillary voltage, 3.00 KV; and source temperature, 350 °C;
Chromatographic conditions: instrument, Thermo U3000; detector, DAD-3000 (RS) (diode array detector); chromatographic column, Shimadzu Inertsil ODS-HL HP 3 µm 3.0×100 mm; flow rate, 0.4 mL/min; column temperature, 30 °C; and mobile phase CH₃OH/H₂O/HCOOH (75/25/0.5).

### HPLC analysis method (I):

Instrument: Thermo U3000; detector: VWD-3×00 (RS) (ultraviolet detector); chromatographic column: Shimadzu Shim-pack VP-ODS 5 µm 4.6×150 mm; flow rate: 0.7 mL/min; column temperature: 30 °C; mobile phase A: CH₃OH/H₂O/AcOH/TEA (65/35/0.1/0.2), mobile phase B: CH₃OH/H₂O/AcOH/TEA (70/30/0.1/0.2); and mobile phase C: CH₃OH/H₂O/AcOH/TEA (50/50/0.1/0.2).

### HPLC analysis method (II):

Instrument: Thermo U3000; detector: VWD-3×00 (RS) (ultraviolet detector); chromatographic column: Shimadzu Shim-pack VP-ODS 5 µm 4.6×150 mm; flow rate: 0.6 mL/min; column temperature: 25 °C; and mobile phase D: CH₃CN/H₂O/HCOOH (65/35/0.3).

### ¹H-NMR analysis method:

¹H-NMR is measured in DMSO-d₆, CDCl₃ and the like using TMS as the internal standard and using BRUKERAVANCE-400MHz or BRUKER FOURIER-300 MHz nuclear magnetic resonance spectrometer at room temperature. The signal peak is expressed as s (singlet), d (doublet), t (triplet), q (quartet), m (multiplet), and dd (double doublet). The unit of the coupling constant (J) is hertz (Hz).

Representative compounds I-1 to 1-20 are prepared in the present invention according to the method described above (see Table 1).

**Table 1 Representative compounds I-1 to 1-20 of the present invention**

| **Compound (Example)** | **Structural formula** | **% purity (HPLC)** | **Retention time (min)** | **Detection wavelength (nm)** | **Mobile phase** | **Analysis method** |
|---|---|---|---|---|---|---|
| **I-1 (22)** | 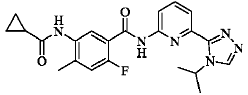 | 98.8 | 17.657 | 295 | C | (I) |
| **I-2 (23)** | 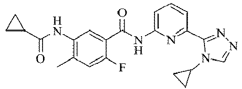 | 95.9 | 4.682 | 232 | A | (I) |
| **I-3 (24)** | 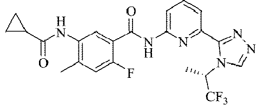 | 90.1 | 6.880 | 232 | A | (I) |
| **I-4 (25)** | 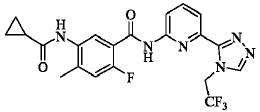 | 92.8 | 4.753 | 233 | B | (I) |
| **I-5 (26)** | 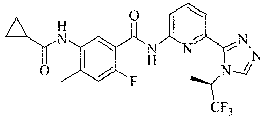 | 99.6 | 4.823 | 232 | B | (I) |
| **I-6 (27)** | 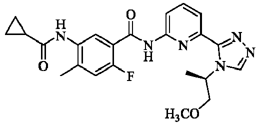 | 97.8 | 17.932 | 231 | B | (I) |
| **I-7 (28)** | 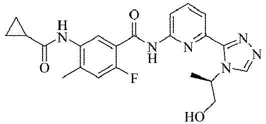 | 88.3 | 6.273 | 311 | C | (I) |
| **I-8 (30)** | 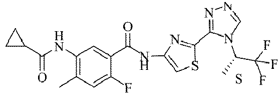 | 95.6 | 6.650 | 232 | A | (I) |
| **I-9 (31)** | 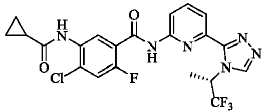 | 90.2 | 5.912 | 231 | B | (I) |
| **I-10 (32)** | 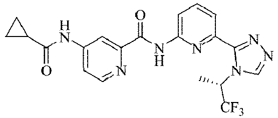 | 99.4 | 4.570 | 230 | D | (II) |
| **I-11 (33)** | 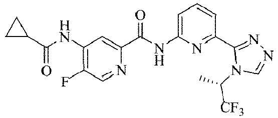 | 96.8 | 4.927 | 230 | D | (II) |
| **I-12 (37)** | 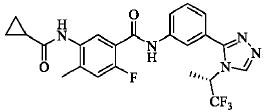 | 99.4 | 5.125 | 230 | A | (I) |
| **I-13 (42)** | 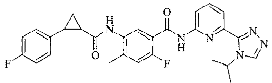 | 98.1 | 14.508 | 230 | A | (I) |
| **I-14 (43)** | 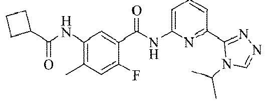 | 99.8 | 6.387 | 230 | A | (I) |
| **I-15 (44)** | 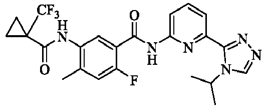 | 97.8 | 7.880 | 230 | A | (I) |
| **I-16 (45)** | 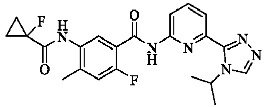 | 98.3 | 4.298 | 230 | B | (I) |
| **I-17 (46)** | 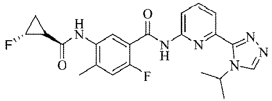 | 92.4 | 5.455 | 232 | A | (I) |
| **1-18 (47)** | 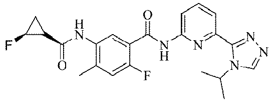 | 93.9 | 4.230 | 232 | A | (I) |
| **1-19 (48)** | 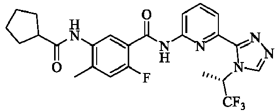 | 98.3 | 7.275 | 232 | B | (I) |
| **1-20 (49)** | 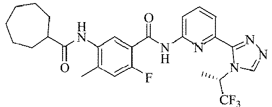 | 97.0 | 12.525 | 232 | B | (I) |

The content of the present invention will be further described below in conjunction with specific examples, but the protection scope of the present invention is not limited to these examples. The percentages as described in the present invention are all weight percentages unless otherwise specified. The numerical ranges described in the description, such as the unit of measurement, reaction condition, physical status of a compound or percentage, are all used to provide clear and correct written references. For those skilled in the art, when implementing the present invention, it is still possible to obtain expected results by using a temperature, concentration, amount, number of carbon atoms or the like outside such ranges or being different from an individual value.

### Example 1 Preparation of intermediate IIa-2: methyl 2-fluoro-4-methyl-5-nitrobenzoate

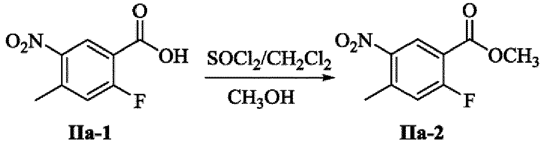

The commercially available **IIa-1** (1.00 g, 5.0 mmol, 1.0 eq) and SOCl₂ (15 mL) were placed into a round bottom flask, heated to 85 °C and refluxed for 2 h, and concentrated to obtain the crude product acyl chloride as a yellow oil, which was directly used in the reaction of the next stage. A solution of this crude product (17.60 g, 50.2 mmol, 1.0 eq) in CH₂Cl₂ (50 mL) was slowly added dropwise to CH₃OH (50 mL), and the resulting solution was stirred at ambient temperature for 30 min, and concentrated to obtain 24.60 g of the crude product **IIa-2** as a light yellow solid, which was directly used in the next reaction.

### Example 2 Preparation of intermediate IIa-3: methyl 5-amino-2-fluoro-4-methylbenzoate

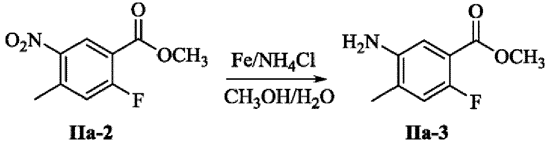

The crude **IIa-2** (24.60 g, 50.2 mmol, 1.0 eq) was dissolved in CH₃OH (200 mL), and water (40 mL), NH₄Cl (13.43 g, 251.0 mmol, 5.0 eq) and Fe powder (11.24 g, 200.8 mmol, 4.0 eq) were added. The resulting mixture was stirred at 75 °C for 2 h, and the completion of the reaction was monitored by LC-MS. After cooling to ambient temperature and filtering, the filtrate was concentrated, and the crude product was separated on a silica gel column (CH₂Cl₂/CH₃OH = 12/1) to obtain 8.05 g (yield 87.7%) of **IIa-3** as a light yellow solid. LC-MS MS-ESI (m/z) 184.1 [M+H]⁺.

### Example 3 Preparation of intermediate IIa-5: methyl 5-(cyclopropylformamido)-2-fluoro-4-methylbenzoate

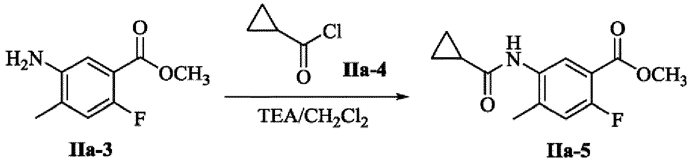

**IIa-3** (8.05 g, 44.0 mmol, 1.0 eq) was dissolved in CH₂Cl₂ (80 mL), TEA (17.78 g, 176.0 mmol, 4.0 eq) was added, and after cooling to 0 °C in an ice/salt bath, the commercially available **IIa-4** (5.5 g, 52.8 mmol, 1.2 eq) was added dropwise. The resulting solution was stirred at ambient temperature for 3 h, and the completion of the reaction was monitored by LC-MS. The reaction solution was diluted by adding CH₂Cl₂ (150 mL) and washed with water once. The aqueous phase was extracted twice with CH₂Cl₂, and the organic phases were combined and concentrated to obtain 11.50 g of the crude product **IIa-5** as a light yellow solid. LC-MS MS-ESI (m/z) 252.1 [M+H]⁺.

### Example 4 Preparation of intermediate IIa: 5-(cyclopropylformamido)-2-fluoro-4-methylbenzoic acid

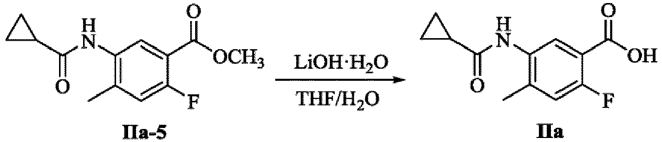

The crude **IIa-5** (11.50 g, 44.0 mmol, 1.0 eq) was dissolved in THF (100 mL), and water (20 mL) and LiOH·H₂O (18.65 g, 444.0 mmol, 10.0 eq) were added. The resulting mixture was stirred at ambient temperature for 16 h, and the completion of the reaction was monitored by TLC. The solvent was concentrated, diluted with water (100 mL), and adjusted to pH 3-4 with 1 N dilute hydrochloric acid. The solid was collected by filtration, and washed once with CH₂Cl₂/CH₃OH (10/1, 100 mL) with stirring, and dried to obtain 9.02 g (yield 86.7%) of **IIa** as a white solid. LC-MS MS-ESI (m/z) 238.1 [M+H]⁺.

### Example 5 Preparation of intermediate IIb-2: methyl 4-chloro-2-fluoro-5-nitrobenzoate

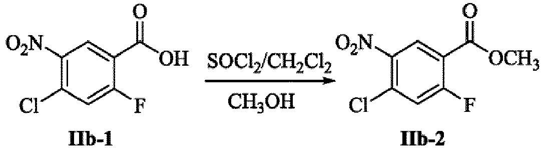

The commercially available **IIb-1** (2.20 g, 10.0 mmol, 1.0 eq) and SOCl₂ (25 mL) were placed into a round bottom flask, heated to 85 °C and refluxed for 2 h, and concentrated to obtain the crude product acyl chloride as a yellow oil, which was directly used in the reaction of the next stage. A solution of this crude product (2.40 g, 10.0 mmol, 1.0 eq) in CH₂Cl₂ (50 mL) was slowly added dropwise to CH₃OH (20 mL), and the resulting solution was stirred at ambient temperature for 30 min, and concentrated to obtain 2.34 g of the crude product **IIb-2** as a light yellow solid, which was directly used in the next reaction.

### Example 6 Preparation of intermediate IIb-3: methyl 5-amino-4-chloro-2-fluorobenzoate

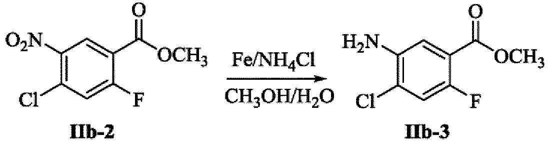

The crude **IIb-2** (2.34 g, 10.0 mmol, 1.0 eq) was dissolved in CH₃OH (20 mL), and water (5 mL), NH₄Cl (2.67 g, 50.0 mmol, 5.0 eq) and Fe powder (2.24 g, 40.0 mmol, 4.0 eq) were added. The resulting mixture was stirred at 75 °C for 2 h, and the completion of the reaction was monitored by TLC. After cooling to ambient temperature and concentrating, the filter cake was washed 5 times with CH₃OH, and the filtrate was concentrated. The crude product was separated on a silica gel column (EtOAc (ethyl acetate/PE (petroleum ether) = 1/2) to obtain 1.33 g (yield 65.5%) of **IIb-3** as a yellow solid. LC-MS MS-ESI (m/z) 204.2 [M+H]⁺.

### Example 7 Preparation of intermediate IIb-5: methyl 4-chloro-5-(cyclopropylformamido)-2-fluorobenzoate

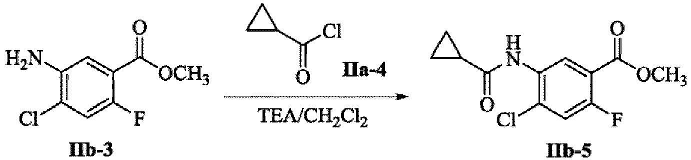

**IIb-3** (1.33 g, 6.5 mmol, 1.0 eq) was dissolved in CH₂Cl₂ (20 mL), TEA (2.64 g, 26.2 mmol, 4.0 eq) was added, and after cooling to 0 °C in an ice/salt bath, the commercially available **IIa-4** (1.02 g, 9.7 mmol, 1.5 eq) was added dropwise. The resulting solution was stirred at ambient temperature for 16 h, and the completion of the reaction was monitored by TLC. The reaction solution was concentrated, and the crude product was separated on a silica gel column (EtOAc/PE = 1/2) to obtain 562.0 mg of **IIb-5** as a yellow solid (yield 31.9%). LC-MS MS-ESI (m/z) 272.2 [M+H]⁺.

### Example 8 Preparation of intermediate IIb: 4-chloro-5-(cyclopropylformamido)-2-fluorobenzoic acid

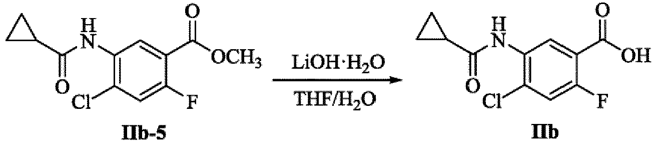

The crude **IIb-5** (562.0 mg, 2.1 mmol, 1.0 eq) was dissolved in THF (100 mL), and water (1 mL) and LiOH·H₂O (868.0 mg, 21.0 mmol, 10.0 eq) were added. The resulting mixture was stirred at ambient temperature for 16 h, and the completion of the reaction was monitored by TLC. The solvent was concentrated, diluted with water (20 mL), and adjusted to pH 3-4 with 1 N dilute hydrochloric acid. The solid was collected by filtration, washed once with CH₂Cl₂/CH₃OH (10/1, 100 mL) with stirring, and dried to obtain 351.2 mg (yield 66.0%) of **IIb** as a white solid. LC-MS MS-ESI (m/z) 258.2 [M+H]⁺.

### Example 9 Preparation of intermediate IIc-5: methyl 4-(cyclopropylformamido)-pyridin-2-formate

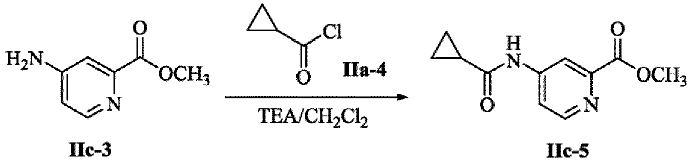

The commercially available **IIc-3** (1.52 g, 10.0 mmol, 1.0 eq) was dissolved in CH₂Cl₂ (20 mL), TEA (4.04 g, 40.0 mmol, 4.0 eq) was added, and after cooling to 0 °C in an ice/salt bath, the commercially available **IIa-4** (1.56 g, 15.0 mmol, 1.5 eq) was added dropwise. The resulting solution was stirred at ambient temperature for 16 h, and the completion of the reaction was monitored by TLC. The reaction solution was concentrated, and the crude product was separated on a silica gel column (EtOAc/PE = 1/2) to obtain 1.56 g (yield 70.9%) of **IIc-5** as a yellow solid. LC-MS MS-ESI (m/z) 221.4 [M+H]⁺.

### Example 10 Preparation of intermediate IIc: 4-(cyclopropylformamido)-pyridin-2-formic acid

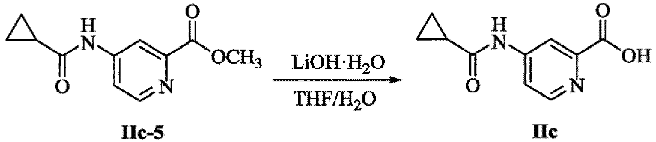

**IIc-5** (1.56 g, 7.1 mmol, 1.0 eq) was dissolved in THF (20 mL), and water (2 mL) and LiOH·H₂O (2.98 g, 70.9 mmol, 10.0 eq) were added. The resulting mixture was stirred at ambient temperature for 16 h. The reaction solution was concentrated, diluted with water (20 mL), adjusted to pH 3-4 with 1 N dilute hydrochloric acid, concentrated until a large amount of solid precipitated. Then the solid was collected by filtration, and dried to obtain 1.35 g (yield 92.5%) of **IIc** as a white solid. LC-MS MS-ESI (m/z) 207.4 [M+H]⁺.

### Example 11 Preparation of intermediate IId-5: methyl 4-(cyclopropylformamido)-5-fluoropyridin-2-formate

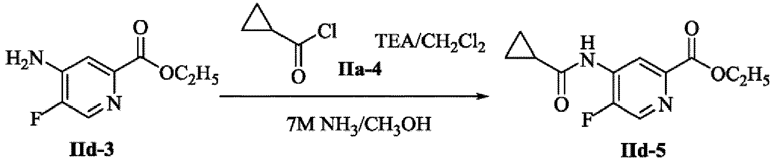

The commercially available **IId-3** (1.84 g, 10.0 mmol, 1.0 eq) was dissolved in CH₂Cl₂ (20 mL), TEA (4.04 g, 40.0 mmol, 4.0 eq) was added, and after cooling to 0 °C in an ice/salt bath, **IIa-4** (1.56 g, 15.0 mmol, 1.5 eq) was added. The resulting solution was stirred at ambient temperature for 16 h, and the reaction was monitored by TLC and product was found to be formed. The reaction solution was concentrated, and the crude product was separated on a silica gel column (EtOAc/PE = 1/1) to obtain 941.0 mg (yield 29.4%) of the bicyclopropylformamido intermediate as a light yellow solid. LC-MS MS-ESI (m/z) 321.2 [M+H]⁺. The intermediate (941.0 mg, 2.9 mmol, 1.0 eq) was dissolved in a solution of 7 M NH₃ in methanol (10 mL), and the resulting solution was stirred at ambient temperature for 1 h, and the completion of the reaction was monitored by LC-MS. The reaction solution was concentrated to obtain 1.27 g of the crude product **IId-5** as a light yellow solid. LC-MS MS-ESI (m/z) 253.3 [M+H]⁺.

### Example 12 Preparation of intermediate lid: 4-(cyclopropylformamido)-5-fluoropyridin-2-formic acid

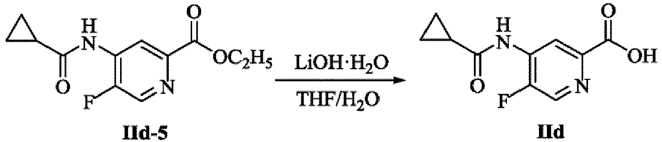

The crude **IId-5** (1.27g, 2.9 mmol, 1.0 eq) was dissolved in THF (15 mL), and water (3 mL) and LiOH·H₂O (1.22g, 2.9 mmol, 10.0 eq) were added. The resulting mixture was stirred at ambient temperature for 2 h, and the completion of the reaction was monitored by TLC. The reaction solution was concentrated, diluted with water (15 mL), and adjusted to pH 3-4 with 1 N dilute hydrochloric acid. The solid was collected by filtration and dried to obtain 494.0 mg (yield 76.1%) of **IId** as a white solid. LC-MS MS-ESI (m/z) 225.2 [M+H]⁺.

### Example 13 Preparation of intermediate IIIa-2: 6-amino-2-pyridineformhydrazide

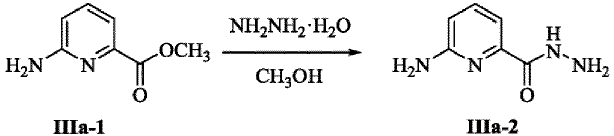

To the commercially available **IIIa-1** (10.00 g, 66.0 mmol, 1.0 eq), methanol (200 mL) and hydrazine hydrate (66.07 g, 132.0 mmol, 2.0 eq) was added sequentially, and heated to reflux for 2 h, and the completion of the reaction was monitored by TLC. The reaction solution was concentrated to remove most of the solvent, filtered off with suction, washed with EtOAc, and dried to obtain 10.20 g of **IIIa-2** as a white solid. LC-MS MS-ESI (m/z) 152.1 [M+H]⁺.

### Example 14 Preparation of intermediate IIIa-3: (E)-N'-(6-(2-((E)-(dimethylamino)methylene)hydrazine-1-carbonyl)pyridin-2-yl)-N,N'-dimethylf ormimide

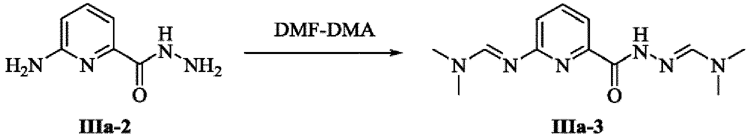

To **IIIa-2** (10.20 g, 66.0 mmol, 1.0 eq), the commercially available DMF-DMA (100 mL) was added, and heated to reflux for 8 h. The completion of the reaction was monitored by TLC. After cooling to room temperature, the reaction solution was concentrated, filtered off with suction, washed with EtOAc and dried to obtain 14.00 g (yield 82.0%) of **IIIa-3** as a yellow solid. LC-MS MS-ESI (m/z) 262.2 [M+H]⁺.

### Example 15 Preparation of intermediate IIIa: 6-(4-isopropyl-4H-1,2,4-triazol-3-yl)pyridin-2-amine

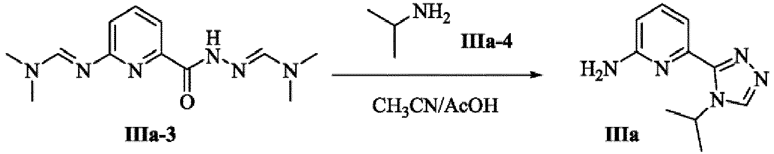

**IIIa-3** (1.00 g, 3.8 mmol, 1.0 eq) and **IIIa-4** (1.3 mL, 15.3 mmol, 4.0 eq) were dissolved in CH₃CN/AcOH (2/1, 30 mL), heated to 95 °C, and reacted for 24 h. The completion of the reaction was monitored by LC-MS. The solvent was concentrated, and the crude product was separated on a silica gel column (CH₂Cl₂/CH₃OH = 10/1) to obtain 490.0 mg (yield 63.3%) of **IIIa** as a viscous solid. LC-MS MS-ESI (m/z) 204.1 [M+H]⁺.

### Example 16 Preparation of intermediate IIIb: 6-(4-cyclopropyl-4H-1,2,4-triazol-3-yl)pyridin-2-amine

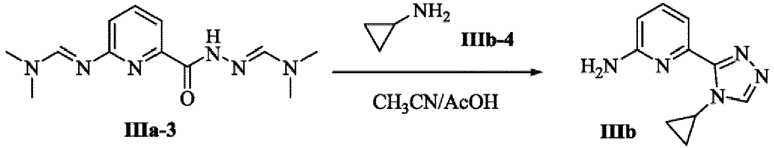

**IIIa-3** (1.00 g, 3.8 mmol, 1.0 eq) and **IIIb-4** (1.1 mL, 15.3 mmol, 4.0 eq) were dissolved in CH₃CN/AcOH (2/1, 30 mL), heated to 95 °C, and reacted for 24 h. The completion of the reaction was monitored by LC-MS. The solvent was concentrated, and the crude product was separated on a silica gel column (CH₂Cl₂/CH₃OH = 10/1) to obtain 620.0 mg (yield 80.8%) of **IIIb** as a viscous solid. LC-MS MS-ESI (m/z) 202.1 [M+H]⁺.

### Example 17 Preparation of intermediate IIIc: (S)-6-(4-(1,1,1-trifluoropropyl-2-yl)-4H-1,2,4-triazol-3-yl)pyridin-2-amine

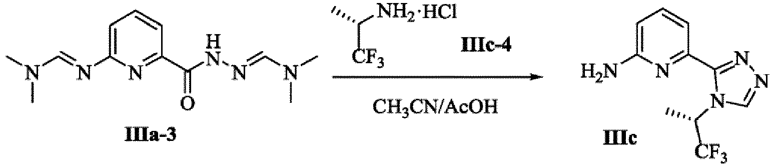

**IIIa-3** (1.00 g, 3.8 mmol, 1.0 eq) and **IIIc-4** (2.27 g, 15.3 mmol, 4.0 eq) was dissolved in CH₃CN/AcOH (2/1, 30 mL), heated to 95 °C, and reacted for 24 h. The completion of the reaction was monitored by LC-MS. The solvent was concentrated, and the crude product was separated on a silica gel column (CH₂Cl₂/CH₃OH = 10/1) to obtain 700.0 mg (yield 71.5%) of **IIIc** as a viscous solid. LC-MS MS-ESI (m/z) 258.2 [M+H]⁺.

### Example 18 Preparation of intermediate IIId: 6-(4-(2,2,2-trifluoroethyl)-4H-1,2,4-triazol-3-yl)pyridin-2-amine

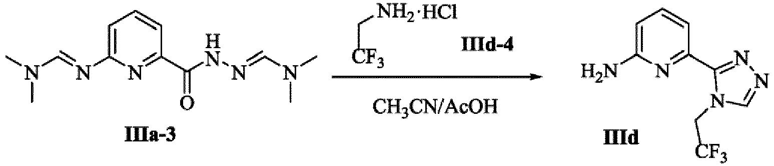

**IIIa-3** (1.00 g, 3.8 mmol, 1.0 eq) and **IIId-4** (2.06 g, 15.3 mmol, 4.0 eq) were dissolved in CH₃CN/AcOH (2/1, 30 mL), heated to 95 °C, and reacted for 24 h. The completion of the reaction was monitored by LC-MS. The solvent was concentrated, and the crude product was separated on a silica gel column (CH₂Cl₂/CH₃OH = 10/1) to obtain 500.0 mg (yield 54.0%) of **IIId** as a viscous solid. LC-MS MS-ESI (m/z) 244.1 [M+H]⁺.

### Example 19 Preparation of intermediate IIIe: (R)-6-(4-(1,1,1-trifluoropropyl-2-yl)-4H-1,2,4-triazol-3-yl)pyridin-2-amine

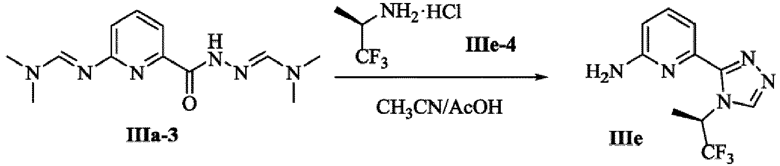

**IIIa-3** (1.00 g, 3.8 mmol, 1.0 eq) and **IIIe-4** (2.27 g, 15.3 mmol, 4.0 eq) were dissolved in CH₃CN/AcOH (2/1, 30 mL), heated to 95 °C, and reacted for 24 h. The completion of the reaction was monitored by LC-MS. The solvent was concentrated, and the crude product was separated on a silica gel column (CH₂Cl₂/CH₃OH = 10/1) to obtain 700.0 mg (yield 71.5%) of **IIIe** as a viscous solid. LC-MS MS-ESI (m/z) 258.2 [M+H]⁺.

### Example 20 Preparation of intermediate IIIf: (R)-6-(4-(1-methoxypropyl-2-yl)-4H-1,2,4-triazol-3-yl)pyridin-2-amine

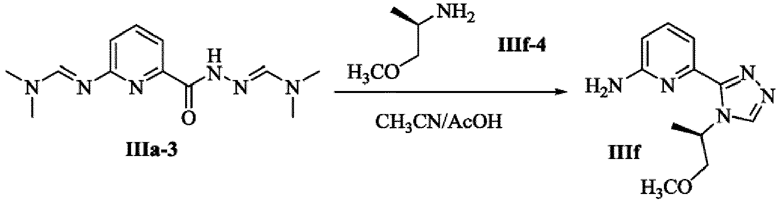

**IIIa-3** (260.0 mg, 1.0 mmol, 1.0 eq) and **IIIf-4** (445.5 mg, 5.0 mmol, 5.0 eq) were dissolved in CH₃CN/AcOH (4/1, 25 mL), and heated to reflux for 24 h. The reaction solution was concentrated, extracted with water, and adjusted to pH 10 with 1 N NaOH solution, extracted 3 times with EtOAc and dried over anhydrous MgSO₄. Then the organic phase was concentrated to obtain 180.0 mg (yield 38.0%) of **IIIf** as a yellow solid. LC-MS MS-ESI (m/z) 233.1 [M+H]⁺.

### Example 21 Preparation of intermediate IIIg: 6-(4-((2R)-1-((tetrahydro-2H-pyran-2-yl)oxa)propyl-2-yl)-4H-1,2,4-triazol-3-yl)pyridin-2-amine

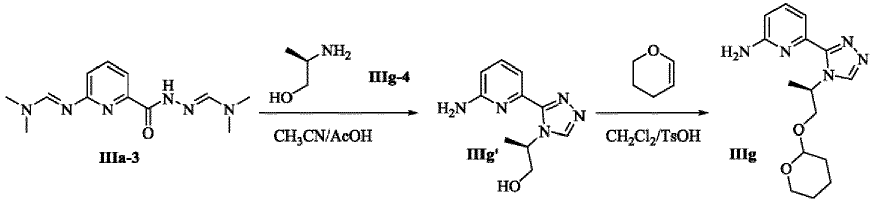

**IIIa-3** (3.00 g, 11.5 mmol, 1.0 eq) and the commercially available **IIIg-4** (3.43 g, 45.8 mmol, 4.0 eq) were dissolved in CH₃CN/AcOH (4/1, 37.5 mL), and the resulting solution was refluxed for 24 h with stirring at 92 °C. The completion of the reaction was monitored by TLC, and then the resultant was cooled to ambient temperature, concentrated, diluted with water, adjusted to pH 8 with 1 N NaOH solution, and concentrated. The resulting solid was suspended in CH₂Cl₂/CH₃OH (10/1, 100 mL) with stirring, then filtered, and the filtrate was concentrated to obtain 7.40 g of the crude product **IIIg'** as a light yellow viscous solid. LC-MS MS-ESI (m/z) 220.4 [M+H]⁺. **IIIg'** (1.10 g, 5.0 mmol, 1.0 eq) was dissolved in CH₂Cl₂ (30 mL), and the commercially available dihydropyran (840.0 mg, 10.0 mmol, 2.0 eq) and p-toluenesulfonic acid (TsOH) (172.0 mg, 1.0 mmol, 0.2 eq) were added. The resulting mixed solution was stirred at ambient temperature for 16 h, and the completion of the reaction was monitored by LC-MS. Then diluted with CH₂Cl₂ (150 mL), and washed once with saturated NaHCO₃ solution (150 mL). The aqueous phase was extracted 7 times with CH₂Cl₂/CH₃OH (10/1, 100 mL), and the organic phases were combined, concentrated, and the crude product was separated on silica gel column (CH₂Cl₂/CH₃OH = 12/1, 6/1, 4/1) to obtain 281.2 mg (yield 18.5%) of **IIIg** as a white solid. LC-MS MS-ESI (m/z) 303.9 [M+H]⁺.

### Example 22: Preparation of the compound of I-1: 5-(cyclopropylformamido)-2-fluoro-4-methyl-N-(6-(4-isopropyl-4H-1,2,4-triazol-3-yl)pyridin-2-yl)benzamide

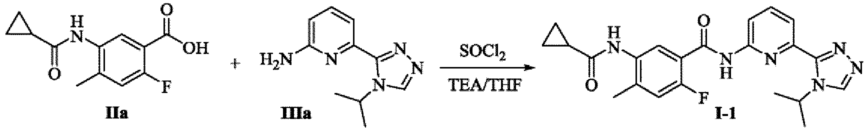

**IIa** (237.0 mg, 1.0 mmol, 1.0 eq) was suspended in SOCl₂ (5 mL), heated to 60 °C, reacted for 15 min until all the raw materials were dissolved, and concentrated to obtain acyl chloride as a yellow solid, which was directly used in the reaction of the next stage. The acyl chloride was dissolved in ultra-dry THF (10 mL), then TEA (0.5 mL) and **IIIa** (102.0 mg, 0.5 mmol, 1.0 eq) were added, and the resulting solution was stirred at 65 °C for 3 h. The completion of the reaction was monitored by LC-MS. After cooling to ambient temperature and concentrating, the crude product was separated by preparative TLC (CH₂Cl₂/CH₃OH = 15/1) to obtain 15.0 mg (yield 7.1%) of **I-1** as a light yellow solid. LC-MS MS-ESI (m/z) 423.0 [M+H]. ¹H-NMR (400 MHz, DMSO-d6) δ ppm 10.7 (s, 1H), 9.67 (s, 1H), 8.85 (s, 1H), 8.18 (d, *J* = 8.2 Hz, 1H), 8.02 (t, *J* = 7.9 Hz, 1H), 7.88 (d, *J* = 7.4 Hz, 1H), 7.76 (d, *J* = 6.9 Hz, 1H), 7.27 (d, *J* = 11.0 Hz, 1H), 5.63-5.66 (m, 1H), 2.29 (s, 3H), 1.88-1.91 (m, 1H), 1.43 (d, *J* = 6.7 Hz, 6H), 0.80-0.86 (m, 4H).

### Example 23 Preparation of the compound of I-2: 5-(cyclopropylformamido)-2-fluoro-4-methyl-N-(6-(4-cyclopropyl-4H-1,2,4-triazol-3-yl)pyridin-2-yl)benzamide

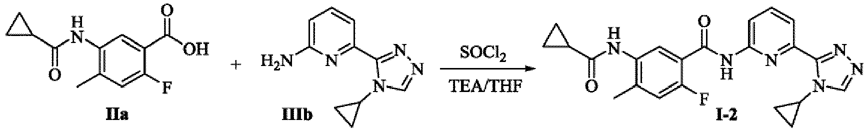

**IIa** (237.0 mg, 1.0 mmol, 1.0 eq) was suspended in SOCl₂ (5 mL), heated to 60 °C, reacted for 15 min until all the raw materials were dissolved, and concentrated to obtain acyl chloride as a yellow solid, which was directly used in the reaction of the next stage. The acyl chloride was dissolved in ultra-dry THF (10 mL), then TEA (0.5 mL) and **IIIb** (100.0 mg, 0.5 mmol, 1.0 eq) were added, and the resulting solution was stirred at 65 °C for 3 h. The completion of the reaction was monitored by LC-MS. After cooling to ambient temperature and concentrating, the crude product was separated by preparative TLC (CH₂Cl₂/CH₃OH = 15/1) to obtain 5.5 mg (yield 2.6%) of I**-2** as an off-white solid. LC-MS MS-ESI (m/z) 421.2 [M+H]⁺. ¹H-NMR (400 MHz, DMSO-d6) δ ppm 10.7 (s, 1H), 9.90 (s, 1H), 8.63 (s, 1H), 8.22 (d, *J* = 7.9 Hz, 1H), 8.02 (t, *J* = 7.8 Hz, 1H), 7.84 (d, *J* = 7.1 Hz, 1H), 7.67 (s, 1H), 7.24 (d, *J* = 10.8 Hz, 1H), 4.17-4.18 (m, 1H), 2.29 (s, 3H), 1.98 (m, 1H), 1.34-1.38 (m, 4H), 1.08-1.13 (m, 4H).

### Example 24: Preparation of the compound of I-3: (S)-5-(cyclopropylformamido)-2-fluoro-4-methyl-N-(6-(4-(1,1,1-trifluoropropyl-2-yl)-4H-1,2,4-tr iazol-3-yl)pyridin-2-yl)benzamide

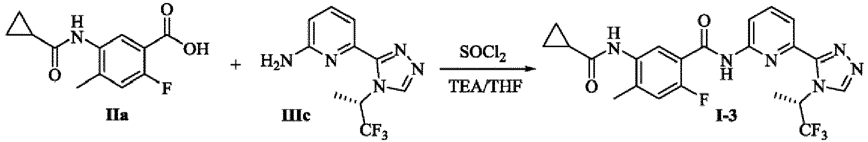

**IIa** (237.0 mg, 1.0 mmol, 1.0 eq) was suspended in SOCl₂ (5 mL), heated to 60 °C, reacted for 15 min until all the raw materials were dissolved, and concentrated to obtain acyl chloride as a yellow solid, which was directly used in the reaction of the next stage. The acyl chloride was dissolved in ultra-dry THF (10 mL), then TEA (0.5 mL) and **IIIc** (130.0 mg, 0.5 mmol, 1.0 eq) were added, and the resulting solution was stirred at 65 °C for 3 h. The completion of the reaction was monitored by LC-MS. After cooling to ambient temperature and concentrating, the crude product was separated by preparative TLC (CH₂Cl₂/CH₃OH = 20/1) to obtain 39.4 mg (yield 16.5%) of **I-3** as an off-white solid. LC-MS MS-ESI (m/z) 477.2 [M+H]⁺. ¹H-NMR (400 MHz, DMSO-d6) δ ppm 10.9 (s, 1H), 9.68 (s, 1H), 9.11 (s, 1H), 8.13 (d, *J* = 7.9 Hz, 1H), 8.04 (t, *J* = 7.9 Hz, 1H), 7.98 (d, *J* = 7.4 Hz, 1H), 7.75 (d, *J* = 6.9 Hz, 1H), 7.29 (d, *J* = 10.8 Hz, 1H), 7.11-7.14 (m, 1H), 2.29 (s, 3H), 1.85-1.89 (m, 1H), 1.80 (d, *J* = 6.9 Hz, 3H), 0.79-0.81 (m, 4H).

### Example 25 Preparation of the compound of I-4: 5-(cyclopropylformamido)-2-fluoro-4-methyl-N-(6-(4-(2,2,2-trifluoroethyl)-4H-1,2,4-triazol-3-yl )pyridin-2-yl)benzamide

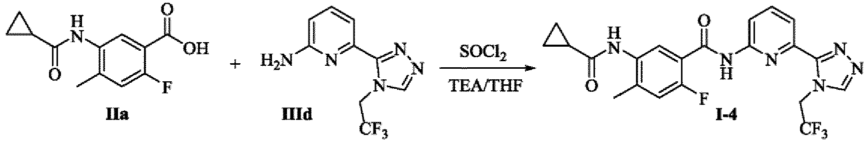

**IIa** (237.0 mg, 1.0 mmol, 1.0 eq) was suspended in SOCl₂ (5 mL), heated to 60 °C, reacted for 15 min until all the raw materials were all dissolved, and concentrated to obtain acyl chloride as a yellow solid, which was directly used in the reaction of the next stage. The acyl chloride was dissolved in ultra-dry THF (10 mL), then TEA (0.5 mL) and **IIId** (121.0 mg, 0.5 mmol, 1.0 eq) were added, and the resulting solution was stirred at 65 °C for 3 h. The completion of the reaction was monitored by LC-MS. After cooling to ambient temperature and concentrating, the crude product was separated by preparative TLC (CH₂Cl₂/CH₃OH = 15/1) to obtain 5.0 mg (yield 2.1%) of **I-4** as a light yellow solid. LC-MS MS-ESI (m/z) 463.2 [M+H]⁺. ¹H-NMR (400 MHz, DMSO-d6) δ ppm 11.0 (s, 1H), 9.70 (s, 1H), 8.77 (s, 1H), 8.17 (d, *J* = 8.2 Hz, 1H), 8.06 (t, *J* = 8.0 Hz, 1H), 7.95 (d, *J* = 7.4 Hz, 1H), 7.75 (d, *J* = 6.8 Hz, 1H), 7.28-7.30 (m, 1H), 5.94-5.97 (m, 2H), 2.30 (s, 3H), 1.88-1.91 (m, 1H), 0.80-0.82 (m, 4H).

### Example 26 Preparation of the compound of I-5: (R)-5-(cyclopropylformamido)-2-fluoro-4-methyl-N-(6-(4-(1,1,1-trifluoropropyl-2-yl)-4H-1,2,4-t riazol-3-yl)pyridin-2-yl)benzamide

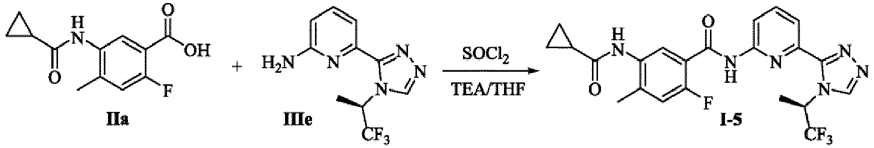

**IIa** (237.0 mg, 1.0 mmol, 1.0 eq) and SOCl₂ (10 mL) were heated to 60 °C, reacted for 15 min until all the raw materials were all dissolved, and concentrated to obtain acyl chloride as a yellow solid, which was directly used in the reaction of the next stage. The acyl chloride was dissolved in ultra-dry THF (10 mL), then TEA (1 mL) and **IIIe** (257.1 mg, 1.0 mmol, 1.0 eq) were added, and the resulting solution was stirred at 65 °C for 2 h. The completion of the reaction was monitored by LC-MS. After cooling to ambient temperature and concentrating, the crude product was separated twice by preparative TLC (CH₂Cl₂/CH₃OH = 12/1, CH₂Cl₂/CH₃OH/HCOOH = 12/1/1) to obtain 48.9 mg (yield 10.3%) of **I-5** as a white solid. LC-MS MS-ESI (m/z) 477.2 [M+H]⁺. ¹H-NMR (400 MHz, DMSO-d6) δ ppm 10.9 (s, 1H), 9.74 (s, 1H), 9.14 (s, 1H), 8.15 (d, *J* = 8.1 Hz, 1H), 8.05 (t, *J* = 8.0 Hz, 1H), 7.98 (d, *J* = 7.6 Hz, 1H), 7.75 (d, *J* = 6.8 Hz, 1H), 7.30 (d, *J* = 10.9 Hz, 1H), 7.12-7.16 (m, 1H), 2.30 (s, 3H), 1.90-1.93 (m, 1H), 1.81 (d, *J* = 7.1 Hz, 3H), 0.80-0.82 (m, 4H).

### Example 27 Preparation of the compound of I-6: (R)-5-(cyclopropylformamido)-2-fluoro-N-6-(4-(1-methoxypropyl-2-yl)-4H-1,2,4-triazol-3-yl)py ridin-2-yl)-4-methylbenzamide

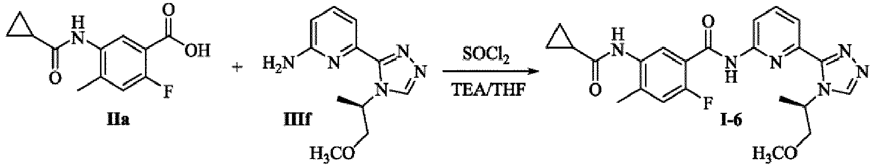

**IIa** (200.0 mg, 0.84 mmol, 1.0 eq) and SOCl₂ (3 mL) were heated to 60 °C, reacted until all solids were dissolved, and concentrated to obtain acyl chloride as a yellow solid, which was directly used in the reaction of the next stage. The acyl chloride was dissolved in ultra-dry THF (10 mL), then TEA (1 mL) and **IIIf** (196.0 mg, 0.84 mmol, 1.0 eq) were added, and the resulting solution was stirred at 65 °C for 2 h. The completion of the reaction was monitored by LC-MS. After cooling to ambient temperature and concentrating, the crude product was separated by preparative TLC (CH₂Cl₂/CH₃OH = 12/1) to obtain 20.0 mg (yield 5.0%) of **I-6** as a white solid. LC-MS MS-ESI (m/z) 453.2 [M+H]⁺. ¹H-NMR (400 MHz, DMSO-d6) δ ppm 10.7 (s, 1H), 9.68 (s, 1H), 8.82 (s, 1H), 8.17 (d, *J* = 8.3 Hz, 1H), 8.02 (t, *J* = 8.1 Hz, 1H), 7.89 (d, *J* = 7.7 Hz, 1H), 7.76 (d, *J* = 7.0 Hz, 1H), 7.27 (d, *J* = 11.0 Hz, 1H), 5.82-5.86 (m, 1H), 3.63-3.68 (m, 1H), 3.51-3.55 (m, 1H), 3.16 (s, 3H), 2.29 (s, 3H), 1.90 (m, 1H), 1.43 (d, *J* = 6.8 Hz, 3H), 0.80-0.85 (m, 4H).

### Example 28 Preparation of the compound of I-7: (R)-5-(cyclopropylformamido)-2-fluoro-N-6-(4-(1-hydroxypropyl-2-yl)-4H-1,2,4-triazol-3-yl)pyr idin-2-yl)-4-methylbenzamide

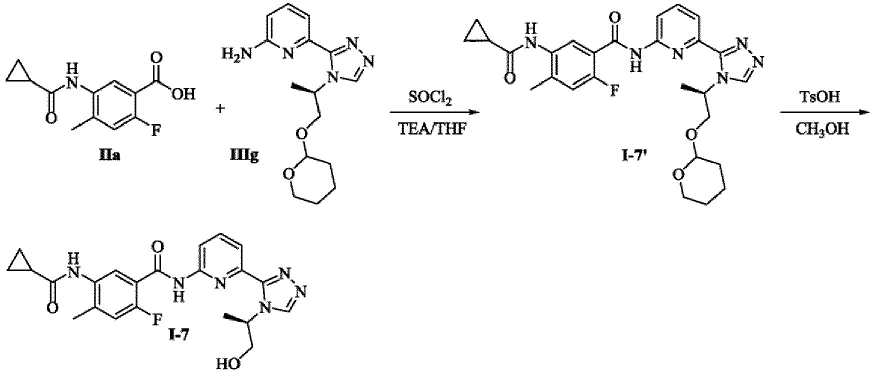

The mixture of **IIa** (203.0 mg, 0.86 mmol, 1.0 eq) and SOCl₂ (10 mL) was heated until the solid was completely dissolved and concentrated to obtain acyl chloride as a yellow solid, which was directly used in the reaction of the next stage. The acyl chloride was dissolved in ultra-dry THF (10 mL), then TEA (1 mL) and **IIIg** (280.0 mg, 0.93 mmol, 1.1 eq) were added, and the resulting solution was stirred at 65 °C for 2.5 h. The completion of the reaction was monitored by LC-MS. After cooling to ambient temperature and concentrating, the crude product was separated by preparative TLC (CH₂Cl₂/CH₃OH = 12/1) to obtain 320.0 mg (yield 71.7%) of orange compound **I-7'.** LC-MS MS-ESI (m/z) 523.3 [M+H]⁺.

**1-7'** (156.6 mg, 0.30 mmol, 1.0 eq) was dissolved in CH₃OH (5 mL), and TsOH (103.2 mg, 0.60 mmol, 2.0 eq) was added. The resulting solution was stirred at ambient temperature for 16 h, and the completion of the reaction was monitored by LC-MS. The reaction solution was concentrated, and the crude product was separated by preparative TLC (CH₂Cl₂/CH₃OH = 12/1) to obtain 34.0 mg (yield 25.9%) of **I-7** as a light yellow solid. LC-MS MS-ESI (m/z) 439.3 [M+H]⁺. ¹H-NMR (400 MHz, DMSO-d6) δ ppm 9.63 (s, 1H), 8.76 (s, 1H), 7.76 (d, *J* = 7.0 Hz, 1H), 7.26 (d, *J* = 6.7 Hz, 1H), 7.20 (s, 1H), 7.14-7.17 (m, 1H), 6.48 (d, *J* = 8.3 Hz, 1H), 6.11 (s, 2H), 5.89-5.93 (m, 1H), 4.58 (d, *J* = 5.3 Hz, 2H), 2.25 (s, 3H), 1.87 (t, *J* = 6.2 Hz, 1H), 1.57 (d, *J* = 7.0 Hz, 3H), 0.81-0.85 (m, 4H).

### Example 29 Preparation of intermediate IIIh: (S)-2-(4-(1,1,1-trifluoropropyl-2-yl)-4H-1,2,4-triazol-3-yl)thiazole-4-amine

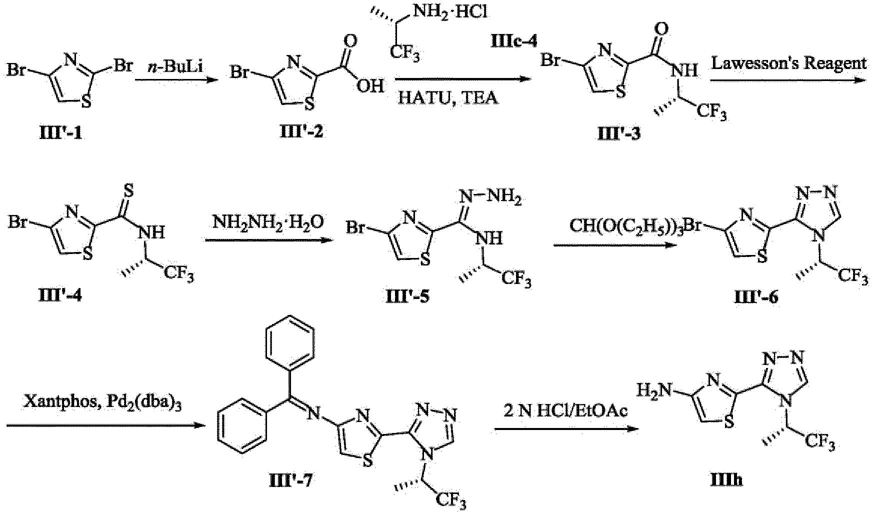

At -70 °C and under the protection of N₂, a solution of n-BuLi in THF (2.5 M, 27.5 mmol, 11 mL, 1.1 eq) was added dropwise to the commercially available **III'-1** (6.00 g, 24.7 mmol, 1.0 eq). The reaction solution was stirred for 1 h while maintaining the temperature at-70 °C, stirred for another 1 h at this low temperature, then CO₂ was introduced, and the reaction solution was stirred for another hour. Then water was added to the reaction solution and ether (Et₂O) was used for extracting. The aqueous layer was adjusted to pH 2 with 2 N dilute hydrochloric acid, and then extracted with EtOAc. The organic layers were combined, dried and concentrated to obtain 4.50 g (yield 87.5%) of **III'-2** as a white solid. LC-MS MS-ESI (m/z) 207.0 [M-H]⁻. ¹H-NMR (300 MHz, DMSO-d₆) δppm 8.22 (s, 1H).

**III'-2** (8.35 g, 40.1 mmol, 1.2 eq) was dissolved in CH₂Cl₂ (100 mL), and HATU (12.7 g, 33.4 mmol, 1.0 eq), the commercially available **IIIc-4** (5.00 g, 33.4 mmol, 1.0 eq) and TEA (10.10 g, 100.0 mmol, 3.0 eq) were added at ambient temperature. After stirring for 3 h, the completion of the reaction was monitored by TLC. The reaction solution was concentrated and the crude product was separated on a silica gel column (PE/EtOAc = 15/1) to obtain 9.60 g (yield 94.8%) of **III'-3** as a white solid. LC-MS MS-ESI (m/z) 302.0 [M-H]⁻. ¹H-NMR (300 MHz, CDCl₃) δppm7.55 (s, 1H), 4.88-4.80 (m, 1H), 1.48 (d, *J* = 7.2 Hz, 3H).

**III'-3** (9.60 g, 31.7 mmol, 1.0 eq) was dissolved in toluene, and Lawesson's Reagent (19.20 g, 47.5 mmol, 1.5 eq) was added at room temperature. The mixture was heated to 120 °C and reacted overnight, and the completion of the reaction was monitored by TLC. The reaction solution was concentrated and the crude product was separated on a silica gel column (PE/EtOAc = 15/1-10/1) to obtain 9.96 g (yield 98.0%) of **III'-4** as a yellow oil. LC-MS MS-ESI (m/z) 318.0 [M-H]⁻. ¹H-NMR (300 MHz, CDCl₃) δppm8.88-8.85 (m, 1H), 7.53 (s, 1H), 5.50-5.42 (m, 1H), 1.55(d,*J* = 6.6 Hz, 3H).

**III'-4** (9.96 g, 31.2 mmol, 1.0 eq) was dissolved in hydrazine hydrate (51.5 g, 1.03 mol, 33.0 eq). The mixture was heated to 125 °C and reacted for 1.5 h, and the completion of the reaction was monitored by TLC. The reaction solution was concentrated and the crude product was separated on a silica gel column (PE/EtOAc = 10/1-3/1) to obtain 4.95 g (yield 50.0%) of **III'-5** as a yellow oil. LC-MS MS-ESI (m/z) 316.1 [M-H]⁻.

**III'-5** (4.95 g, 15.6 mmol, 1.0 eq) was dissolved in C₂H₅OH (50 mL), CH(OC₂H₅)₃ (11.6 g, 78.0 mmol, 5.0 eq) and catalytic amount of concentrated H₂SO₄ (0.05 mL) were added at room temperature. The mixture was maintained at room temperature and reacted for 2 h, and the completion of the reaction was monitored by TLC. The reaction solution was concentrated and the crude product was separated on a silica gel column (PE/EtOAc = 15/1-3/1) to obtain 5.00 g (yield 98.0%) of **III'-6** as a yellow oil. LC-MS MS-ESI (m/z) 328.1 [M+H]⁺. ¹H-NMR (300 MHz, CDCl₃) δppm8.45 (s, 1H), 7.41 (s,1H), 6.54-6.45 (m, 1H), 1.82 (d,*J* = 7.2 Hz,3H).

**III'-6** (5.00 g, 15.3 mmol, 1.0 eq) was dissolved in 1,4-dioxane and water (4/1, 75 mL), and benzophenone imine (5.54 g, 30.6 mmol, 2.0 eq), K₂CO₃ (4.22 g, 30.6 mmol, 2.0 eq), Pd₂(dba)₃ (1.40 g, 1.54 mmol, 0.1 eq) and Xantphos (3.24 g, 7.64 mmol, 0.5 eq) were added under N₂ protection. The mixture was heated to 100 °C and reacted overnight, and the completion of the reaction was monitored by TLC. The reaction solution was diluted with water (30 mL) and extracted 3 times with EtOAc. The organic phases were combined and the crude product was separated on a silica gel column (PE/EtOAc = 10/1-1/3) to obtain 3.00 g (yield 46.0%) of **III'-7** as a yellow oil. LC-MS MS-ESI (m/z) 428.1 [M+H]⁺. ¹H-NMR (300 MHz, CDCl₃) δppm8.34 (s, 1H), 7.80-7.78 (m, 2H), 7.53 (d, *J* = 0.6 Hz, 1H), 7.47-7.44 (m, 2H), 7.38-7.28 (m, 3H), 7.21-7.18 (m, 2H), 6.68 (s, 1H), 6.05-5.97 (m, 1H),1.60 (d, *J* = 7.2 Hz, 3H).

**III'-7** (3.00 g, 3.8 mmol, 1.0 eq) was dissolved in 2 N dilute hydrochloric acid and EtOAc (50 mL), and the mixture was stirred overnight at room temperature. The completion of the reaction was monitored by TLC. The reaction solution was concentrated and the crude product was separated by preparative HPLC to obtain 0.45 g (yield 35.0%) of **IIIh** as a yellow solid. LC-MS MS-ESI (m/z) 264.1 [M+H]⁺. ¹H-NMR (300 MHz, CDCl₃) δppm9.27 (s, 1H), 7.20 (s, 1H), 6.48-6.41 (m, 1H), 1.83 (d,*J* = 7.2 Hz, 3H).

### Example 30 Preparation of the compound of I-8: (S)-5-(cyclopropylformamido)-2-fluoro-4-methyl-N-2-(4-(1,1,1-trifluoropropyl-2-yl)-4H-1,2,4-tri azol-3-yl)thiazol-4-yl)benzamide

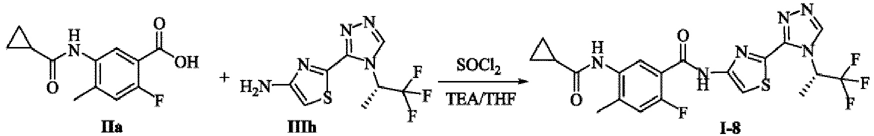

The mixture of **IIa** (100.0 mg, 0.42 mmol, 1.0 eq) and SOCl₂ (6 mL) was heated until the solid was completely dissolved, and concentrated to obtain acyl chloride as a yellow solid, which was directly used in the reaction of the next stage. The acyl chloride was dissolved in ultra-dry THF (10 mL), then TEA (1 mL) and home-made **IIIh** hydrochloride (50.0 mg, 0.17 mmol, 0.4 eq) were added, and the resulting solution was stirred at 65 °C for 2 h. The completion of the reaction was monitored by LC-MS. After cooling to ambient temperature and concentrating, the crude product was separated 3 times by preparative TLC (CH₂Cl₂/CH₃OH = 12/1, CH₂Cl₂/CH₃OH/HCOOH = 12/1/1, EtOAc/PE = 2/1) to obtain 18.5 mg (yield 9.2%) of I**-8** as a white solid. LC-MS MS-ESI (m/z) 482.2 [M+H]⁺. ¹H-NMR (400 MHz, DMSO-d6) δ ppm 11.4 (s, 1H), 9.67 (s, 1H), 9.22 (s, 1H), 7.95 (s, 1H), 7.73 (d, *J* = 7.0 Hz, 1H), 7.26 (d, *J* = 11.0 Hz, 1H), 6.62 (t, *J* = 7.8 Hz, 1H), 2.27 (s, 3H), 1.88-1.91 (m, 1H), 1.83 (d, *J* = 7.2 Hz, 3H), 0.80 (d, *J* = 6.1 Hz, 4H).

### Example 31 Preparation of the compound of I-9: (S)-4-chloro-5-(cyclopropylformamido)-2-fluoro-N-(6-(4-(1,1,1-trifluoropropyl-2-yl)-4H-1,2,4-tr iazol-3-yl)pyridin-2-yl)benzamide

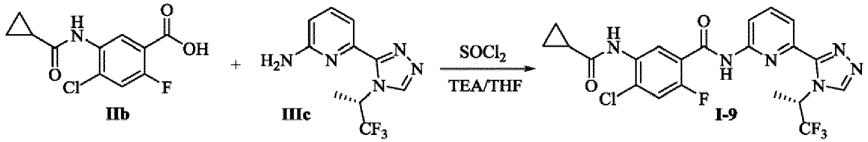

The mixture of **IIb** (130.0 mg, 0.5 mmol, 2.0 eq) and SOCl₂ (6 mL) was heated until the solid was completely dissolved, and concentrated to obtain acyl chloride as a yellow solid, which was directly used in the reaction of the next stage. The acyl chloride was dissolved in ultra-dry THF (10 mL), then TEA (1 mL) and **IIIc** (65.0 mg, 0.3 mmol, 1.0 eq) were added, and the resulting solution was stirred at 65 °C for 3 h. The completion of the reaction was monitored by LC-MS. After concentrating, the crude product was separated by preparative TLC (CH₂Cl₂/CH₃OH = 20/1) to obtain 6.0 mg (yield 4.8%) of **I-9** as a light yellow solid. LC-MS MS-ESI (m/z) 497.1 [M+H]⁺.

### Example 32 Preparation of the compound of I-10: (S)-4-(cyclopropylformamido)-N-(6-(4-(1,1,1-trifluoropropyl-2-yl)-4H-1,2,4-triazol-3-yl)pyridin-2-yl)pyridin-2-formamide

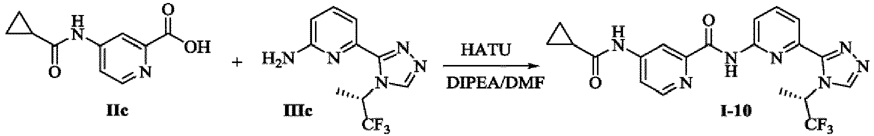

**IIc** (154.5 mg, 0.8 mmol, 1.5 eq) was dissolved in ultra-dry DMF (5 mL), then HATU (406.2 mg, 1.3 mmol, 2.5 eq), DIPEA (258.0 mg, 2.0 mmol, 4.0 eq) and **IIIc** (128.5 mg, 0.5 mmol, 1.0 eq) were added, and the resulting solution was stirred at ambient temperature for 16 h. The reaction was quenched by adding water (50 mL), and extracted 3 times with EtOAc (50 mL). The organic phases were combined, washed 3 times with saturated brine (100 mL), dried over anhydrous Na₂SO₄, and concentrated. The crude product was separated twice by TLC (CH₂Cl₂/CH₃OH = 12/1, CH₂Cl₂/CH₃OH/HCOOH = 24/1/1) to obtain 10.9 mg (yield 4.9%) of **I-10** as a white solid. LC-MS MS-ESI (m/z) 446.2 [M+H]⁺. ¹H-NMR (400 MHz, DMSO-d6) δ ppm 11.0 (s, 1H), 10.93 (s, 1H), 9.14 (s, 1H), 8.61 (d, *J* = 5.5 Hz, 1H), 8.39 (s, 1H), 8.23 (d, *J* = 8.2 Hz, 1H), 8.08 (t, *J* = 7.9 Hz, 1H), 7.97 (d, *J* = 7.6 Hz, 1H), 7.92 (d, *J* = 5.4 Hz, 1H), 7.01-7.08 (m, 1H), 1.83 (d, *J* = 7.0 Hz, 3H), 1.24 (s, 1H), 0.81-0.93 (m, 4H).

### Example 33 Preparation of the compound of I-11: (S)-4-(cyclopropylformamido)-5-fluoro-N-(6-(4-(1,1,1-trifluoropropyl-2-yl)-4H-1,2,4-triazol-3-yl )pyridin-2-yl)pyridin-2-formamide

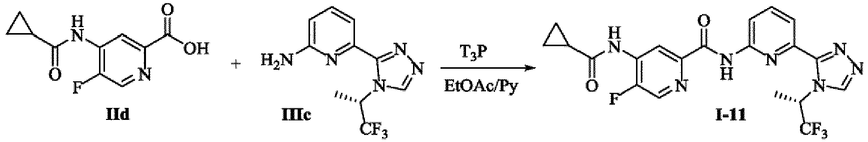

**lid** (33.6 mg, 0.3 mmol, 1.5 eq) and **IIIc** (52.0 mg, 0.2 mmol, 1.0 eq) were dissolved in EtOAc/Py (2/1, 12 mL), and the reaction solution was cooled to below 5 °C in an ice-water bath, and a solution of T₃P (250.0 mg, 0.4 mmol, 2.0 eq) in 50% EtOAc was added dropwise. Then, after stirring for 3 h at room temperature, the completion of the reaction was monitored by TLC. The reaction solution was diluted with water (50 mL) and extracted twice with EtOAc. The organic phases were combined, and washed once with saturated sodium bicarbonate followed by once with saturated brine, and the organic phase was dried and concentrated. The crude product was separated by preparative TLC (CH₂Cl₂/CH₃OH = 15 /1) to obtain 7.0 mg (yield 7.5%) of **I-11** as a white solid. LC-MS MS-ESI (m/z) 464.2 [M+H]⁺. ¹H-NMR (400 MHz, DMSO-d6) δ ppm 10.9 (s, 1H), 10.73 (s, 1H), 9.14 (s, 1H), 9.09 (d, *J* = 6.5 Hz, 1H), 8.73 (d, *J* = 2.4 Hz, 1H), 8.22 (d, *J* = 8.3 Hz, 1H), 8.08 (t, *J* = 8.0 Hz, 1H), 7.97 (d, *J* = 7.6 Hz, 1H), 7.00-7.07 (m, 1H), 2.18-2.23 (m, 1H), 1.83 (d, *J* = 7.1 Hz, 3H), 0.91-0.93 (m, 4H).

### Example 34 Preparation of intermediate IVa-2: methyl 3-(5-(cyclopropylformamido)-2-fluoro-4-methylbenzamide)benzoate

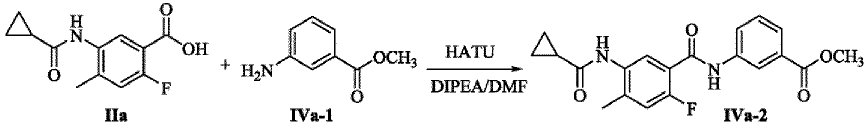

**IIa** (948.0 mg, 4.0 mmol, 1.0 eq) and the commercially available **IVa-1** (604.0 mg, 4.0 mmol, 1.0 eq) were dissolved in DMF (20 mL), then DIPEA (2.6 M, 16.0 mmol, 4.0 eq) was added, and then HATU (2.28 g, 6.0 mmol, 1.5 eq) was added all at once. The reaction solution was stirred overnight at room temperature, and the completion of the reaction was monitored by LC-MS. EtOAc (60 mL) was added to the reaction solution, washed 3 times with water followed by once with saturated brine, the organic phase was dried and concentrated, and the crude product was separated by column chromatography (EtOAc/PE = 1/1) to obtain 450.0 mg (yield 30.4%) of **IVa-2** as a white solid. LC-MS MS-ESI (m/z) 371.3 [M+H]⁺, 741.3 [2M+H]⁺.

### Example 35 Preparation of intermediate IVa-3: 3-(5-(cyclopropylformamido)-2-fluoro-4-methylbenzamide)benzohydrazide

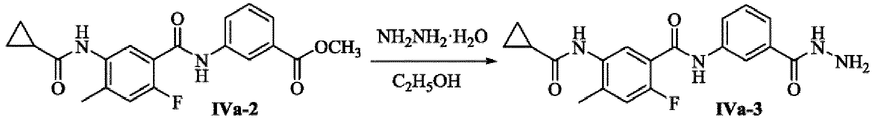

**IVa-2** (450.0 mg, 1.2 mmol, 1.0 eq) was dissolved in C₂H₅OH (15 mL), and hydrazine hydrate (4.12 g, 82.2 mmol, 68.0 eq) was added. After heating to 85°C and reacting for 3h, a white solid was precipitated. The reaction solution was cooled to room temperature and filtered off with suction. The filter cake was washed with EtOAc, and dried to obtain 320.0 mg (yield 71.1%) of **IVa-3** as a white solid. LC-MS MS-ESI (m/z) 371.2 [M+H]⁺.

### Example 36 Preparation of intermediate IVa-4: (E)-5-(cyclopropylformamido)-N-(3-(2-((dimethylamino)methylene)hydrazine-1-carbonyl)pheny l)-2-fluoro-4-methylbenzamide

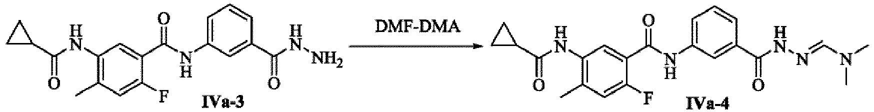

**IVa-3** (320.0 mg, 0.86 mmol, 1.0 eq) was suspended in DMF-DMA (10 mL), heated to 100°C, reacted for 3 h, and the completion of the reaction was monitored by TLC. A white solid was precipitated, filtered off with suction, and the filter cake was washed with EtOAc and dried to obtain 350.0 mg (yield 95.5%) of **IVa-4** as a white solid. LC-MS MS-ESI (m/z) 426.2 [M+H]⁺.

### Example 37 Preparation of the compound of I-12: (S)-5-(cyclopropylformamido)-2-fluoro-4-methyl-N-(3-(4-(1,1,1-trifluoropropyl-2-yl)-4H-1,2,4-tr iazol-3-yl)phenyl)benzamide

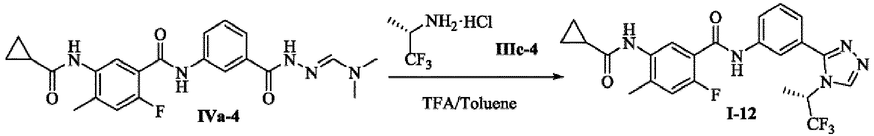

**IVa-4** (106.0 mg, 0.3 mmol, 1.0 eq) and **IIIc-4** (150.0 mg, 1.0 mmol, 3.3 eq) were suspended in toluene (15 mL), and 2 drops of trifluoroacetic acid (TFA) was added. After heating to 110°C and reacting for 12 h, the completion of the reaction was monitored by LC-MS. The reaction solution was concentrated, dissolved with EtOAc (60 mL), and washed twice with 0.1 N dilute hydrochloric acid followed by once with saturated brine. The organic phase was dried and concentrated, and the crude product was separated by preparative TLC (CH₂Cl₂/CH₃OH = 15/1) to obtain 9.0 mg (yield 7.5%) of I**-12** as a white solid. LC-MS MS-ESI (m/z) 476.2 [M+H]⁺. ¹H-NMR (400 MHz, DMSO-d6) δ ppm 10.5 (s, 1H), 9.69 (s, 1H), 9.07 (s, 1H), 7.97 (s, 1H), 7.90 (d, *J* = 7.7 Hz, 1H), 7.72 (d, *J* = 6.0 Hz, 1H), 7.56 (t, *J* = 7.7 Hz, 1H), 7.34 (d, *J* = 7.1 Hz, 1H), 7.26 (d, *J* = 10.5 Hz, 1H), 5.18-5.21 (m, 1H), 2.28 (s, 3H), 1.89 (s, 1H), 1.78 (d, *J* = 6.3 Hz, 3H), 0.79-0.80 (m, 4H).

### Example 38 Preparation of intermediate Va-1: 2-fluoro-N-(6-(4-isopropyl-4H-1,2,4-triazol-3-yl)pyridin-2-yl)-4-methyl-5-nitrobenzamide

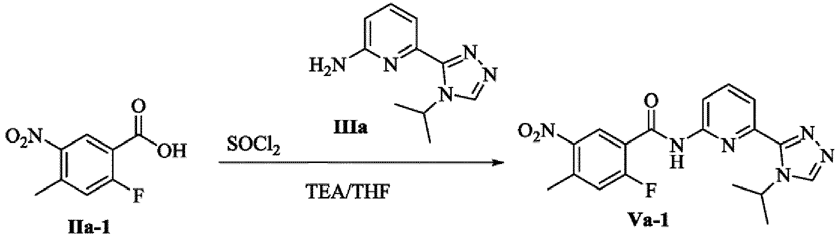

The commercially available **IIa-1** (1.00 g, 5.0 mmol, 1.0 eq) and SOCl₂ (15 mL) were placed into a round bottom flask, heated to 85 °C and refluxed for 2 h, and concentrated to obtain the crude product acyl chloride as a yellow oil, which was directly used in the reaction of the next stage. (The yield is calculated as 100%). This crude product (1.72 g, 5.0 mmol, 1.0 eq) was dissolved in ultra-dry THF (20 mL), then TEA (2.03 g, 20.1 mmol, 4.0 eq) and **IIIa** (1.02 g, 5.0 mmol, 1.0 eq) were added, and the resulting mixture was stirred at 65°C for 3 h. The completion of the reaction was monitored by LC-MS. After cooling to ambient temperature, the solid was collected by filtration, washed once with EtOAc (15 mL) with stirring, and dried to obtain 1.18 g (yield 62.0%) of **Va-1** as an off-white solid. LC-MS MS-ESI (m/z) 385.2 [M+H]⁺.

### Example 39 Preparation of intermediate Va: 5-amino-2-fluoro-N-(6-(4-isopropyl-4H-1,2,4-triazol-3-yl)pyridin-2-yl)-4-methylbenzamide

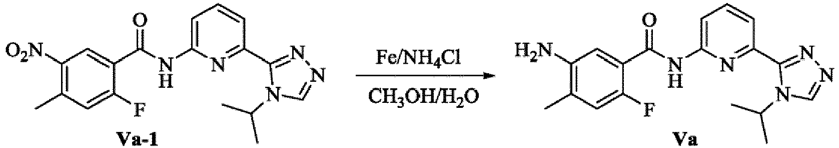

**Va-1** (1.18 g, 3.1 mmol, 1.0 eq) was dissolved in CH₃OH (20 mL) and water (4 mL), and NH₄Cl (819.0 mg, 15.3 mmol, 5.0 eq) and Fe powder (685.0 mg, 12.2 mmol, 4.0 eq) were added. The resulting mixture was stirred at 75 °C for 3 h, and the completion of the reaction was monitored by LC-MS. After cooling to ambient temperature and filtering, the filtrate was concentrated, and the obtained solid was washed once with CH₂Cl₂ (15 mL) with stirring and dried to obtain 1.03 g (yield 95.3%) of **Va** as a gray solid. LC-MS MS-ESI (m/z) 355.3 [M+H]⁺.

### Example 40 Preparation of intermediate Vb-1: (S)-2-fluoro-4-methyl-5-nitro-N-(6-(4-(1,1,1-trifluoropropyl-2-yl)-4H-1,2,4-triazol-3-yl)pyridin-2 -yl)benzamide

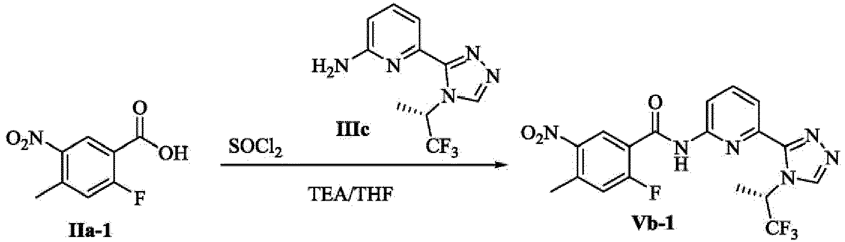

The commercially available **IIa-1** (1.99 g, 10.0 mmol, 1.0 eq) was added to SOCl₂ (20 mL). The resulting solution was refluxed at 85°C for 2h and concentrated, and after adding ultra-dry THF (20 mL), concentrated again to obtain the intermediate acyl chloride. The acyl chloride was dissolved in ultra-dry THF (20 mL), then TEA (2.5 mL) and **IIIc** (1.12 g, 4.4 mmol, 0.4 eq) were added, and the resulting solution was stirred at 65°C for 2 h. The completion of the reaction was monitored by LC-MS. After cooling to ambient temperature and concentrating, the crude product was separated by silica gel column (CH₂Cl₂/CH₃OH = 12/1) to obtain 662.0 mg (yield 34.4%) of **Vb-1** as a yellow viscous solid. LC-MS MS-ESI (m/z) 439.2 [M+H]⁺.

### Example 41 Preparation of intermediate Vb: (S)-5-amino-2-fluoro-4-methyl-N-(6-(4-(1,1,1-trifluoropropyl-2-yl)-4H-1,2,4-triazol-3-yl)pyridin -2-yl)benzamide

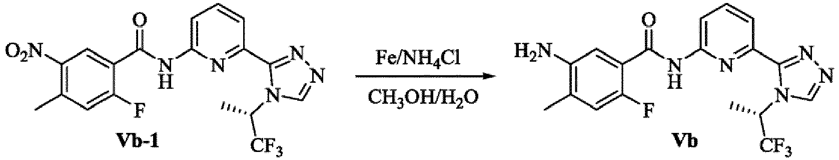

**Vb-1** (662.0 mg, 1.5 mmol, 1.0 eq) was dissolved in CH₃OH (15 mL), and water (3 mL), NH₄Cl (404.0 mg, 7.6 mmol, 5.0 eq) and Fe powder (338.2 mg, 6.0 mmol, 4.0 eq) were added. The resulting mixture was stirred at 75 °C for 2 h, and the completion of the reaction was monitored by LC-MS. After filtering, the filter cake was washed 5 times with CH₃OH, and the filtrate was concentrated, and the crude product was separated by silica gel column (CH₂Cl₂/CH₃OH = 12/1) to obtain 342.0 mg (yield 55.5%) of **Vb** as a yellow solid. LC-MS MS-ESI (m/z) 409.3 [M+H]⁺.

### Example 42 Preparation of the compound of I-13: 2-fluoro-5-(2-(4-fluorophenyl)cyclopropyl-1-formamido)-N-(6-(4-isopropyl-4H-1,2,4-triazol-3-yl )pyridin-2-yl)-4-methylbenzamide

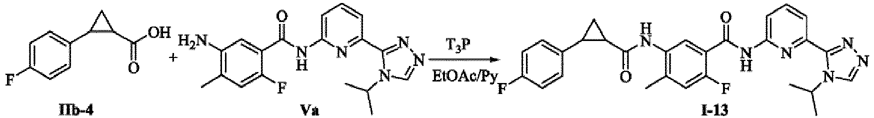

The commercially available **IIb-4** (72.0 mg, 0.4 mmol, 2.0 eq) and **Va** (72.0 mg, 0.2 mmol, 1.0 eq) were dissolved in EtOAc (8 mL) and Py (4 mL), and after cooling to 0 °C in an ice/salt bath, a solution of T₃P in 50% EtOAc (0.3 mL) was added dropwise. The resulting solution was stirred at ambient temperature for 5 h, and the completion of the reaction was monitored by LC-MS. The reaction solution was diluted with EtOAc (40 mL) and washed once with 1 N dilute hydrochloric acid. The aqueous phase was extracted twice with EtOAc, and the organic phases were combined, dried over anhydrous Na₂SO₄, and concentrated. The crude product was separated by preparative TLC (CH₂Cl₂/CH₃OH = 6/1) to obtain 16.0 mg (yield 15.5%) of **I-13** as a white solid. LC-MS MS-ESI (m/z) 517.3 [M+H]⁺. ¹H-NMR (400 MHz, DMSO-d6) δ ppm 10.7 (s, 1H), 9.71 (s, 1H), 8.85 (s, 1H), 8.18 (d, *J*= 8.2 Hz, 1H), 8.04 (t, *J*=8.0 Hz, 1H), 7.85-7.90 (m, 2H), 7.23-7.28 (m, 3H),7.13 (t, *J*= 8.7 Hz, 2H), 5.61-5.66 (m, 1H), 2.39-2.43 (m,1H), 2.29 (s, 3H), 2.19-2.21 (m,1H), 1.46-1.50 (m, 1H), 1.44 (d, *J*= 6.6 Hz, 6H), 1.33-1.37 (m, 1H).

### Example 43 Preparation of the compound of I-14: 5-(cyclobutylformamido)-2-fluoro-N-(6-(4-isopropy1-4H-1,2,4-triazol-3-yl)pyridin-2-yl)-4-methy lbenzamide

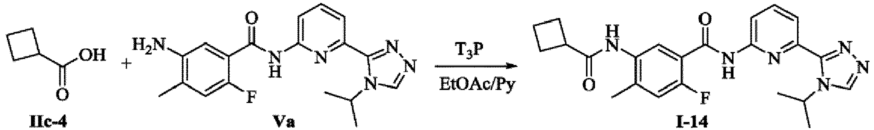

The commercially available **IIc-4** (30.0 mg, 0.3 mmol, 1.5 eq) and **Va** (72.0 mg, 0.2 mmol, 1.0 eq) were dissolved in EtOAc (12 mL) and Py (6 mL), and after cooling to 0 °C in an ice/salt bath, a solution of T₃P in 50% EtOAc (0.3 mL) was added dropwise. The resulting solution was stirred at ambient temperature for 5 h, and the completion of the reaction was monitored by LC-MS. The reaction solution was diluted with CH₂Cl₂ (50 mL) and washed once with 1 N dilute hydrochloric acid. The aqueous phase was extracted 7 times with CH₂Cl₂/CH₃OH (10/1), and the organic phases were combined, dried over anhydrous Na₂SO₄, and concentrated. The crude product was separated by preparative TLC (CH₂Cl₂/CH₃OH = 12/1) to obtain 36.0 mg (yield 34.9%) of **I-14** as an off-white solid. LC-MS MS-ESI (m/z) 437.2 [M+H]⁺. ¹H-NMR (400 MHz, DMSO-d6) δ ppm 10.7 (s, 1H), 9.25 (s, 1H), 8.85 (s, 1H), 8.18 (d, *J* = 8.2 Hz, 1H), 8.02 (t, *J* = 7.8 Hz, 1H), 7.88 (d, *J* = 7.6 Hz, 1H), 7.70 (d, *J* = 6.9 Hz, 1H), 7.26 (d, *J* = 11.0 Hz, 1H), 5.63-5.66 (m, 1H), 2.24 (s, 3H), 2.19-2.21 (m, 2H), 2.13-2.15 (m, 2H), 1.94-1.96 (m, 2H), 1.81-1.84 (m, 1H), 1.44 (d, *J* = 6.7 Hz, 6H).

### Example 44: Preparation of the compound of I-15: 2-fluoro-N-(6-(4-isopropyl-4H-1,2,4-triazol-3-yl)pyridin-2-yl)-4-methyl-5-(1-(trifluoromethyl)cy clopropyl-1-formamido)benzamide

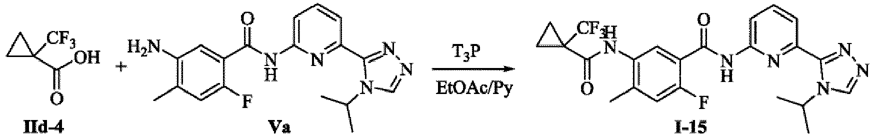

The commercially available **IId-4** (47.0 mg, 0.3 mmol, 1.5 eq) and **Va** (72.0 mg, 0.2 mmol, 1.0 eq) were dissolved in EtOAc (12 mL) and Py (6 mL), and after cooling to 0 °C in an ice/salt bath, a solution of T₃P in 50% EtOAc (0.3 mL) was added dropwise. The resulting solution was stirred at ambient temperature for 5 h, and the completion of the reaction was monitored by LC-MS. The reaction solution was diluted with CH₂Cl₂ (50 mL) and washed once with 1 N dilute hydrochloric acid. The aqueous phase was extracted 7 times with CH₂Cl₂/CH₃OH (10/1), and the organic phases were combined, dried over anhydrous Na₂SO₄, and concentrated. The crude product was separated by preparative TLC (CH₂Cl₂/CH₃OH = 12/1) to obtain 14.0 mg (yield 13.6%) of **I-15** as an off-white solid. LC-MS MS-ESI (m/z) 491.2 [M+H]⁺. ¹H-NMR (400 MHz, DMSO-d6) δ ppm 10.7 (s, 1H), 9.42 (s, 1H), 8.85 (s, 1H), 8.18 (d, *J* = 8.0 Hz, 1H), 8.02 (t, *J* = 7.4 Hz, 1H), 7.89 (d, *J* = 7.4 Hz, 1H), 7.52 (d, *J* = 6.4 Hz, 1H), 7.31 (d, *J* = 10.9 Hz, 1H), 5.66-5.63 (m, 1H), 2.22 (s, 3H), 1.52-1.50 (m, 2H), 1.44 (d, *J* = 6.4 Hz, 6H), 1.35-1.33 (m, 2H).

### Example 45 Preparation of the compound of I-16: 2-fluoro-N-(6-(4-isopropyl-4H-1,2,4-triazol-3-yl)pyridin-2-yl)-4-methyl-5-(1-(fluoro)cyclopropyl -1-formamido)benzamide

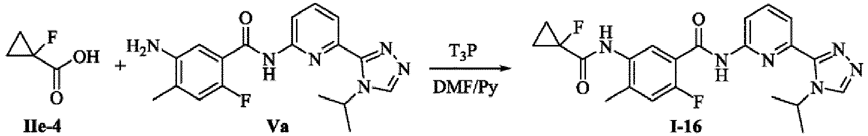

The commercially available **IIe-4** (44 mg, 0.4 mmol, 1.5 eq) and **Va** (88.1 mg, 0.2 mmol, 1.0 eq) were dissolved in anhydrous DMF/Py (2/1, 6 mL), and a solution of T₃P in 50% EtOAc (0.3 mL) was added dropwise under a condition of an ice-water bath. The completion of the reaction was monitored by TLC. The reaction solution was diluted with CH₂Cl₂/CH₃OH (10/1, 20 mL) and extracted with water. The organic phases were combined, washed with water, saturated sodium bicarbonate and saturated brine, and dried over anhydrous sodium sulfate. The solvent was concentrated, and the crude product was separated by column chromatography (CH₂Cl₂/CH₃OH = 20/1) to obtain 15.0 mg of **I-16** as a yellow viscous solid. LC-MS MS-ESI (m/z) 441.2 [M+H]⁺. ¹H-NMR (400 MHz, DMSO-d6) δ ppm 10.8 (s, 1H), 10.01 (s, 1H), 8.86 (s, 1H), 8.19 (d, *J* = 8.1 Hz, 1H), 8.02 (t, *J* = 8.1 Hz, 1H), 7.88 (d, *J* = 7.3 Hz, 1H), 7.60 (d, *J* = 6.8 Hz, 1H), 7.32 (d, *J* = 11.1 Hz, 1H), 5.64-5.67 (m, 1H), 2.27 (s, 3H), 1.44 (d, *J* = 6.7 Hz, 6H), 1.39-1.41 (m, 2H), 1.27-1.33 (m, 2H).

### Example 46 Preparation of the compound of I-17: 2-fluoro-5-((1R,2R)-2-fluorocyclopropyl-1-formamido)-N-(6-(4-isopropyl-4H-1,2,4-triazol-3-yl) pyridin-2-yl)-4-methylbenzamide

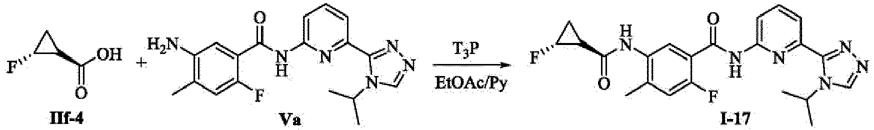

The commercially available **IIf-4** (31.2 mg, 0.3 mmol, 1.5 eq) and **Va** (71.0 mg, 0.2 mmol, 1.0 eq) were dissolved in EtOAc (8 mL) and Py (4 mL), and after cooling to below 5 °C in an ice-water bath, a solution of T₃P in 50% EtOAc (0.3 mL) was added dropwise. The resulting solution was stirred at ambient temperature for 3 h, and the completion of the reaction was monitored by LC-MS. The reaction solution was diluted with water (50 mL). Then the aqueous phase was extracted twice with EtOAc, and the organic phases were combined, washed with saturated sodium bicarbonate and saturated brine, dried over anhydrous Na₂SO₄, and concentrated. The crude product was separated by preparative TLC (CH₂Cl₂/CH₃OH = 15/1) to obtain 19.0 mg (yield 21.5%) of **I-17** as an off-white solid. LC-MS MS-ESI (m/z) 441.2 [M+H]⁺. ¹H-NMR (400 MHz, DMSO-d6) δ ppm 10.7 (s, 1H), 9.93 (s, 1H), 8.86 (s, 1H), 8.18 (d, *J* = 8.2 Hz, 1H), 8.02 (t, *J* = 8.0 Hz, 1H), 7.88 (d, *J* = 7.5 Hz, 1H), 7.77 (d, *J* = 6.9 Hz, 1H), 7.28 (d, *J* = 11.0 Hz, 1H), 5.63-5.66 (m, 1H), 4.79-4.96 (m, 1H), 2.30 (s, 3H), 1.48-1.54 (m, 1H), 1.45 (d, *J* = 6.8 Hz, 6H), 1.20-1.25 (m, 2H).

### Example 47 Preparation of the compound of I-18: 2-fluoro-5-((1R,2S)-2-fluorocyclopropyl-1-formamido)-N-(6-(4-isopropyl-4H-1,2,4-triazol-3-yl)p yridin-2-yl)-4-methylbenzamide

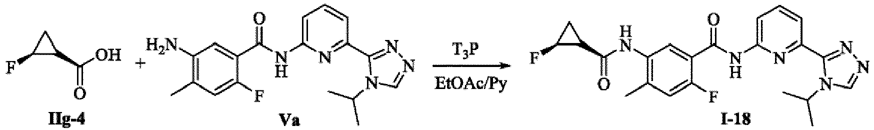

The commercially available **IIg-4** (31.2 mg, 0.3 mmol, 1.5 eq) and **Va** (71.0 mg, 0.2 mmol, 1.0 eq) were dissolved in EtOAc (8 mL) and Py (4 mL), and after cooling to below 5 °C in an ice-water bath, a solution of T₃P in 50% EtOAc (0.3 mL) was added dropwise. The resulting solution was stirred at ambient temperature for 3 h, and the completion of the reaction was monitored by LC-MS. The reaction solution was diluted with water (50 mL). Then the aqueous phase was extracted twice with EtOAc, and the organic phases were combined, washed with saturated sodium bicarbonate and saturated brine, dried over anhydrous Na₂SO₄, and concentrated. The crude product was separated by preparative TLC (CH₂Cl₂/CH₃OH = 15/1) to obtain 18.0 mg (yield 20.4%) of **I-18** as an off-white solid. LC-MS MS-ESI (m/z) 441.2 [M+H]⁺. ¹H-NMR (400 MHz, DMSO-d6) δ ppm 10.7 (s, 1H), 9.94 (s, 1H), 8.85 (s, 1H), 8.18 (d, *J* = 8.2 Hz, 1H), 8.02 (t, *J* = 7.9 Hz, 1H), 7.88 (d, *J* = 7.6 Hz, 1H), 7.72 (d, *J* = 6.8 Hz, 1H), 7.27 (d, *J* = 11.0 Hz, 1H), 5.61-5.68 (m, 1H), 4.85-5.02 (m, 1H), 2.28 (s, 3H), 1.57-1.65 (m, 1H), 1.43 (d, *J* = 6.6 Hz, 6H), 1.07-1.23 (m, 2H).

### Example 48 Preparation of the compound of I-19: (S)-5-(cyclopentylformamido)-2-fluoro-4-methyl-N-(6-(4-(1,1,1-trifluoropropyl-2-yl)-4H-1,2,4-tr iazol-3-yl)pyridin-2-yl)benzamide

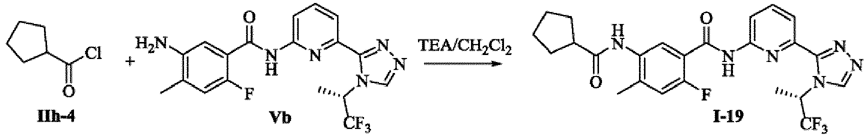

**Vb** (170.0 mg, 0.4 mmol, 1.0 eq) was dissolved in CH₂Cl₂ (10 mL), TEA (1 mL) was added, and after cooling to 0 °C in an ice/salt bath, the commercially available **IIh-4** (83.2 mg, 0.6 mmol, 1.5 eq) was added. The resulting solution was stirred at ambient temperature for 4 h, and the completion of the reaction was monitored by LC-MS. The reaction solution was concentrated, and the crude product was separated twice by preparative TLC (CH₂Cl₂/CH₃OH = 12/1, EtOAc/CH₃OH = 6/1) to obtain 50.2 mg (yield 23.7%) of **I-19** as a white solid. LC-MS MS-ESI (m/z)505.2 [M+H]⁺. ¹H-NMR (400 MHz, DMSO-d6) δ ppm 10.9 (s, 1H), 9.40 (s, 1H), 9.12 (s, 1H), 8.14 (d, *J* = 8.2 Hz, 1H), 8.05 (t, *J* = 7.9 Hz, 1H), 7.99 (d, *J* = 7.5 Hz, 1H), 7.68 (d, *J* = 6.9 Hz, 1H), 7.30 (d, *J* = 10.9 Hz, 1H), 7.12-7.15 (m, 1H), 2.84-2.88 (m, 1H), 2.26 (s, 3H), 1.87-1.89 (m, 2H), 1.81 (d, *J* = 7.1 Hz, 3H), 1.67-1.75 (m, 4H), 1.55-1.66 (m, 2H).

### Example 49 Preparation of the compound of I-20: (S)-5-(cycloheptylformamido)-2-fluoro-4-methyl-N-(6-(4-(1,1,1-trifluoropropyl-2-yl)-4H-1,2,4-tr iazol-3-yl)pyridin-2-yl)benzamide

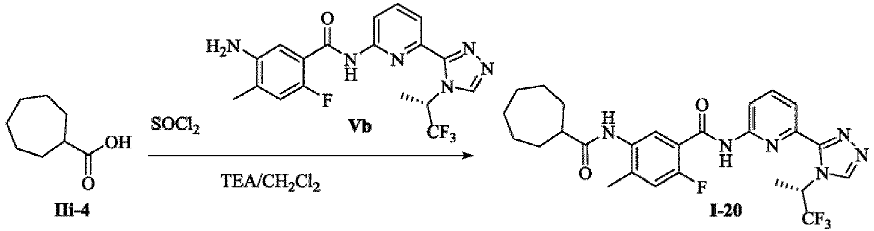

The commercially available **IIi-4** (160.1 mg, 1.0 mmol, 1.0 eq) was added to SOCl₂ (5 mL). The resulting mixture was refluxed for 1 h with stirring at 85°C and concentrated, and after adding ultra-dry THF (10 mL), concentrated again to obtain the intermediate acyl chloride as a yellow oil. Vb (80.0 mg, 0.2 mmol, 0.2 eq) was dissolved in CH₂Cl₂ (10 mL), TEA (0.5 mL) was added, and after cooling to 0 °C in an ice/salt bath, the intermediate acyl chloride was added. The resulting solution was stirred at ambient temperature for 4 h. Then the reaction solution was concentrated, and the crude product was separated twice by preparative TLC (CH₂Cl₂/CH₃OH = 10/1, CH₂Cl₂/CH₃OH/HCOOH = 24/1/1) to obtain 3.8 mg (yield 3.6%) of **I-20** as a light yellow solid. LC-MS MS-ESI (m/z)533.2 [M+H]⁺. ¹H-NMR (400 MHz, DMSO-d6) δ ppm 10.9 (s, 1H), 9.34 (s, 1H), 9.12 (s, 1H), 8.14 (d, *J* = 8.0 Hz, 1H), 8.05 (t, *J* = 7.9 Hz, 1H), 7.98 (d, *J* = 7.5 Hz, 1H), 7.64 (d, *J* = 6.9 Hz, 1H), 7.29 (d, *J* = 10.9 Hz, 1H), 7.12-7.15 (m, 1H), 2.54-2.60 (m, 1H), 2.25 (s, 3H), 1.86-1.91 (m, 2H), 1.81 (d, *J* = 7.1 Hz, 3H), 1.46-1.75 (m, 12H).

### In vitro biological evaluation

This detection method is used to evaluate the inhibitory activity of in vitro protein level binding of the compound of the present invention.

The purpose of this detection is to comprehensively evaluate the inhibitory activity of different compounds against ASK1 kinase.

### Example A Enzymatic inhibition screening method of ASK1 in vitro

In this detection, homogeneous time resolved fluorescence (HTRF) was used to evaluate the inhibitory level of the compound on the enzyme activity of recombinant human ASK1 in an in vitro reaction system.

### The main principle of the experiment

The basic principle of the detection of enzymatic activity in vitro: a specific substrate labeled with biotin at the end is phosphorylated under the action of a kinase, and the reaction product is mixed with the EU3+-Cryptate-labeled antibody that recognizes the phosphorylation site and XL665-labeled streptavidin. When the two fluorescent molecules are bound to the substrate at the same time, Eu will produce 620 nM fluorescence under the stimulation of external excitation light (320 nm), and meanwhile, XL665 will be excited by energy transfer to produce 665 nm fluorescence. The phosphorylation of the substrate is evaluated by comparing the changes of fluorescence at two wavelengths (620 nm and 665 nm). The inhibition of kinase activity by different test compounds, when added, is reflected in the changes in the degree of phosphorylation of the substrate, which shows different fluorescence signal ratios (665/620), and thereby the inhibitory activity of the compounds against the kinases can be calculated. The basic detection principle is known in the prior art **(**Cisbio, Nature Method 2006, June 23; DOI: 10.1038/NMETH883**).**

### The main process of the experiment

Human recombinant ASK1 (MAP3K5) kinase, 2× kinase reaction buffer, and ATP (10 mM) were purchased from Invitrogen (Cat. No.: PR7349B), and HTRF detection kit, HTRF KinEASE STK discovery kit, was purchased from Cisbio (Cat. No.: 62ST0PEB).

The experimental process was carried out in accordance with the procedures required by the detection reagent manual (https://www.cisbio.com/sites/default/files/ressources/cisbio_dd_pi_62ST0PEB.pdf). The details are as follows:
(1) Experimental preparation: the kinase reaction buffer (working solution) is prepared as required and used to dilute the test compound into different concentration gradients (the maximum concentration of the compound is 4 µM).
(2) 10 µL of the enzymatic reaction system (including 2.5 µL of test compound, 5 µL of kinase reaction buffer and 2.5 µL of ATP solution (provided in the kit)) is mixed well and reacted at room temperature for 1 h. The enzymatic reaction is carried out in a 96-well microplate.
(3) EU3+-Cryptate-labeled antibody and XL665-labeled streptavidin are diluted with reaction termination buffer in an appropriate ratio, and 5 µL of each of two diluted detection solutions is added to each reaction well and reacted at room temperature for 2 h.
(4) The reaction is set up with a control reaction at the same time, including a positive control without test compound and a negative control without ASK1 kinase. All detections are carried out in duplicate.
(5) After the reaction, a fluorescence detector (TecanSPARK 10M) is used to detect the fluorescence signal of each well, wherein, the excitation wavelength is 320 nm, and the detected emission wavelengths are 620 nm and 665 nm, respectively.
(6) The 665/620 ratio of each well is calculated respectively, and the 665/620 ratio of the negative control well is subtracted from that of the positive control well to get the basic level of phosphorylation of the substrate. The formula for calculating the enzymatic inhibition rate of the test compound: inhibition rate (%) = 1-(ratio of detection wells-ratio of negative wells)/basic level of phosphorylation of the substrate. The phosphorylation inhibition rates are calculated for the test compounds with different concentration gradients, and then the 50% enzymatic inhibiting concentration (IC₅₀) is calculated by using the IC₅₀ calculator. The summary data of the representative compounds of the present invention are as follows (see Table 2).

**Table 2 ASK1 enzymatic inhibition rate of the representative compounds of the present invention detected by HTRF method (single concentration 100 nM)**

| **Compound** | **Inhibition Rate %** | **Compound** | **Inhibition Rate %** | **Compound** | **Inhibition Rate %** |
|---|---|---|---|---|---|
| **I-1** | 54 | **I-3** | 51 | **I-4** | 32 |
| **I-6** | 13 | **I-8** | 24 | **I-9** | 33 |
| **I-10** | 44 | **I-11** | 27 | **I-13** | 18 |
| **I-14** | 42 | **I-16** | 21 | **I-17** | 15 |
| **I-18** | 34 | **I-19** | 52 | **I-20** | 16 |

It can be seen from the above results that the representative compounds of the present invention has good activity of inhibiting the enzymatic activity of ASK1 *in vitro.*

## Claims

1. A compound of formula I, 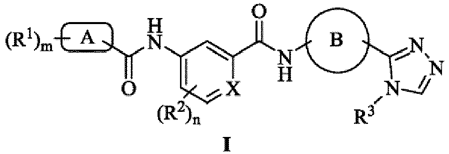 wherein,
R¹ is one or more same or different substituents independently selected from H, halogen, CN, C₁-C₄ alkyl, C₁-C₄ haloalkyl, NH₂, COOH, C₁-C₄ alkylamino, C₁-C₄ alkyloxy and Ar¹;
wherein, Ar¹ is selected from a benzene ring and a pyridine ring, wherein the benzene ring and the pyridine ring can be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
R² is one or more same or different substituents independently selected from H, halogen, CN, C₁-C₄ alkyl and C₁-C₄ haloalkyl;
R³ is C₁-C₄ alkyl, C₃-C₆ cycloalkyl, C₁-C₄ haloalkyl, halo C₃-C₆ cycloalkyl, cyano substituted C₁-C₄ alkyl, C₃-C₆ heterocycloalkyl, hydroxy substituted C₁-C₄ alkyl or C₁-C₄ alkoxy substituted C₁-C₄ alkyl;
X is selected from C and N;
A is selected from C₃-C₇ cycloalkyl and C₃-C₇ heterocycloalkyl;
B is an aromatic ring,
preferably selected from a benzene ring, a pyridine ring, a thiazole ring, a furan ring, a thiophene ring, a pyrrole ring, a pyrazole ring, a oxazole ring, a isoxazole ring and a quinoline ring, wherein the aromatic ring can be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
m is an integer from 1 to 5; and
n is an integer from 1 to 4;
or a prodrug, a stereoisomer, a pharmaceutically acceptable salt, a hydrate or other solvates thereof.

2. The compound of formula I according to claim 1,
wherein,
R¹ is one or more same or different substituents independently selected from H, halogen, CN, C₁-C₄ alkyl, C₁-C₄ haloalkyl, NH₂, COOH, C₁-C₄ alkylamino, C₁-C₄ alkyloxy and Ar¹;
wherein, Ar¹ is selected from a benzene ring and a pyridine ring, wherein the benzene ring and the pyridine ring can be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
R² is one or more same or different substituents independently selected from H, halogen, CN and C₁-C₄ alkyl;
R³ is C₁-C₄ alkyl, C₃-C₆ cycloalkyl, C₁-C₄ haloalkyl, halo C₃-C₆ cycloalkyl, cyano substituted C₁-C₄ alkyl, C₃-C₆ heterocycloalkyl, hydroxy substituted C₁-C₄ alkyl or C₁-C₄ alkoxy substituted C₁-C₄ alkyl;
X is selected from C and N;
A is selected from C₃-C₇ cycloalkyl and C₃-C₇ heterocycloalkyl;
B is an aromatic ring,
preferably selected from a benzene ring, a pyridine ring, a thiazole ring, a furan ring, a thiophene ring, a pyrrole ring, a pyrazole ring, a oxazole ring, a isoxazole ring and quinoline, wherein the aromatic ring can be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
m is an integer from 1 to 5; and
n is an integer from 1 to 4.

3. The compound of formula I according to claim 1,
wherein,
R¹ is one or more same or different substituents independently selected from H, halogen, CN, C₁-C₄ alkyl, C₁-C₄ haloalkyl, NH₂, COOH, C₁-C₄ alkylamino and C₁-C₄ alkyloxy;
R² is one or more same or different substituents independently selected from H, halogen, CN and C₁-C₄ alkyl;
R³ is C₁-C₄ alkyl, C₃-C₆ cycloalkyl, C₁-C₄ haloalkyl, halo C₃-C₆ cycloalkyl, cyano substituted C₁-C₄ alkyl, C₃-C₆ heterocycloalkyl, hydroxy substituted C₁-C₄ alkyl or C₁-C₄ alkoxy substituted C₁-C₄ alkyl;
X is selected from C and N;
A is selected from C₃-C₇ cycloalkyl and C₃-C₇ heterocycloalkyl;
B is an aromatic ring,
preferably selected from a benzene ring, a pyridine ring, a thiazole ring, a furan ring, a thiophene ring, a pyrrole ring, a pyrazole ring, a oxazole ring, a isoxazole ring and a quinoline ring, and the aromatic ring can be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
m is an integer from 1 to 5; and
n is an integer from 1 to 4.

4. The compound of formula I according to claim 1,
wherein,
R¹ is one or more same or different substituents independently selected from H, halogen, CN, C₁-C₄ alkyl, C₁-C₄ haloalkyl, NH₂, COOH, C₁-C₄ alkylamino and C₁-C₄ alkyloxy;
R² is one or more same or different substituents independently selected from H, halogen, CN and C₁-C₄ alkyl;
R³ is C₁-C₄ alkyl, C₃-C₆ cycloalkyl, C₁-C₄ haloalkyl, halo C₃-C₆ cycloalkyl or cyano substituted C₁-C₄ alkyl;
X is selected from C and N;
A is selected from C₃-C₇ cycloalkyl and C₃-C₇ heterocycloalkyl;
B is an aromatic ring,
preferably selected from a benzene ring, a pyridine ring, a thiazole ring, a furan ring, a thiophene ring, a pyrrole ring, a pyrazole ring, a oxazole ring, a isoxazole ring and a quinoline ring, and the aromatic ring can be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
m is an integer from 1 to 5; and
n is an integer from 1 to 4.

5. The compound of formula I according to claim 1,
wherein,
R¹ is one or more same or different substituents independently selected from H, halogen, CN, C₁-C₄ alkyl, C₁-C₄ haloalkyl, NH₂, COOH, C₁-C₄ alkylamino and C₁-C₄ alkyloxy;
R² is one or more same or different substituents independently selected from H, halogen, CN and C₁-C₄ alkyl;
R³ is C₁-C₄ alkyl, C₃-C₆ cycloalkyl, C₁-C₄ haloalkyl, halo C₃-C₆ cycloalkyl or cyano substituted C₁-C₄ alkyl;
X is selected from C and N;
A is selected from C₃-C₅ cycloalkyl and C₃-C₅ heterocycloalkyl;
B is an aromatic ring preferably selected from a benzene ring, a pyridine ring and a thiazole ring, and the aromatic ring can be substituted by one or more same or different substituents independently selected from H, halogen, CN, C₁-C₄ alkyl, C₁-C₄ haloalkyl, NH₂, C₁-C₄ alkylamino and C₁-C₄ alkyloxy;
m is an integer from 1 to 5; and
n is an integer from 1 to 4.

6. The compound of formula I according to claim 1,
wherein,
R¹ is one or more same or different substituents independently selected from H, halogen, CN and C₁-C₄ alkyl;
R² is one or more same or different substituents independently selected from H, halogen, CN and methyl;
R³ is C₁-C₄ alkyl, C₃-C₆ cycloalkyl, C₁-C₄ haloalkyl, halo C₃-C₆ cycloalkyl or cyano substituted C₁-C₄ alkyl;
X is selected from C and N;
A is selected from C₃-C₄ cycloalkyl and C₃-C₄ heterocycloalkyl;
B is an aromatic ring,
preferably selected from a benzene ring, a pyridine ring and a thiazole ring, and the aromatic ring can be substituted by one or more same or different substituents independently selected from H, halogen, CN, C₁-C₄ alkyl and C₁-C₄ haloalkyl;
m is an integer from 1 to 5; and
n is an integer from 1 to 4.

7. The compound of formula I according to claim 1,
wherein,
R¹ is one or more same or different substituents independently selected from H, halogen and CN;
R² is one or more same or different substituents independently selected from H, F, Cl, CN and methyl;
R³ is C₁-C₄ alkyl, C₃-C₆ cycloalkyl, C₁-C₄ haloalkyl, halo C₃-C₆ cycloalkyl or cyano substituted C₁-C₄ alkyl;
X is selected from C and N;
A is selected from C₃-C₄ cycloalkyl and C₃-C₄ heterocycloalkyl;
B is an aromatic ring,
preferably selected from a benzene ring, a pyridine ring and a thiazole ring, and the aromatic ring can be substituted by one or more same or different substituents independently selected from H, halogen, CN, methyl and CF₃;
m is an integer from 1 to 5; and
n is an integer from 1 to 4.

8. The compound of formula I according to claim 1,
wherein,
R¹ is H;
R² is one or more same or different substituents independently selected from H, F, Cl, CN and methyl;
R³ is C₁-C₄ alkyl, C₃-C₆ cycloalkyl, C₁-C₄ haloalkyl, halo C₃-C₆ cycloalkyl and cyano substituted C₁-C₄ alkyl;
X is selected from C and N;
A is selected from C₃-C₄ cycloalkyl;
B is an aromatic ring preferably selected from a benzene ring, a pyridine ring and a thiazole ring, and the aromatic ring can be substituted by one or more same or different substituents independently selected from H, halogen, CN, methyl and CF₃;
m is 1; and
n is an integer from 1 to 3.

9. The compound of formula I according to claim 1,
wherein,
R¹ is H;
R² is one or more same or different substituents independently selected from H, F, Cl, CN and methyl;
R³ is C₁-C₄ alkyl, C₃-C₆ cycloalkyl, C₁-C₄ haloalkyl, halo C₃-C₆ cycloalkyl or cyano substituted C₁-C₄ alkyl;
X is selected from C and N;
A is selected from C₃-C₄ cycloalkyl;
B is an aromatic ring preferably selected from a benzene ring, a pyridine ring and a thiazole ring, and the aromatic ring can be substituted by one or more same or different substituents independently selected from H, halogen, CN, methyl and CF₃;
m is 1; and
n is an integer from 1 to 2.

10. The compound of formula I according to claim 1, wherein the compound of formula I is selected from the group consisting of:
5-(cyclopropylformamido)-2-fluoro-4-methyl-*N*-(6-(4-isopropyl-4*H*-1,2,4-triazol-3-yl)pyridi n-2-yl)benzamide;
5-(cyclopropylformamido)-2-fluoro-4-methyl-*N*-(6-(4-cyclopropyl-4*H*-1,2,4-triazol-3-yl)pyr idin-2-yl)benzamide;
(*S*)-5-(cyclopropylformamido)-2-fluoro-4-methyl-*N*-(6-(1,1-trifluoropropyl-2-yl)-4*H*-1, 2,4-triazol-3-yl)pyridin-2-yl)benzamide;
5-(cyclopropylformamido)-2-fluoro-4-methyl-*N*-(6-(4-(2,2,2-trifluoroethyl)-4*H*-1,2,4-triazol -3-yl)pyridin-2-yl)benzamide;
(*R*)-5-(cyclopropylformamido)-2-fluoro-4-methyl-*N*-(6-(4-(1,1-trifluoropropyl-2-yl)-4*H*-1, 2,4-triazol-3-yl)pyridin-2-yl)benzamide;
(*R*)-5-(cyclopropylformamido)-2-fluoro-*N*-6-(4-(1-methoxypropyl-2-yl)-4*H*-1,2,4-triazol-3-yl)pyridin-2-yl)-4-methylbenzamide;
(*R*)-5-(cyclopropylformamido)-2-fluoro-*N*-6-(4-(1-hydroxypropyl-2-yl)-4*H*-1,2,4-triazol-3-y l)pyridin-2-yl)-4-methylbenzamide;
(*S*)-5-(cyclopropylformamido)-2-fluoro-4-methyl-*N*-2-(4-(1,1,1-trifluoropropyl-2-yl)-4*H*-1,2 ,4-triazol-3-yl)thiazol-4-yl)benzamide;
(*S*)-4-chloro-5-(cyclopropylformamido)-2-fluoro-*N*-(6-(4-(1,1,1-trifluoropropyl-2-yl)-4*H*-1, 2,4-triazol-3-yl)pyridin-2-yl)benzamide;
(*S*)-4-(cyclopropylformamido)-*N*-(6-(4-(1,1,1-trifluoropropyl-2-yl)-4*H*-1,2,4-triazol-3-yl)pyr idin-2-yl)pyridin-2-formamide;
(*S*)-4-(cyclopropylformamido)-5-fluoro-*N*-(6-(4-(1,1,1-trifluoropropyl-2-yl)-4*H*-1,2,4-triazol -3-yl)pyridin-2-yl)pyridin-2-formamide;
(*S*)-5-(cyclopropylformamido)-2-fluoro-4-methyl-*N*-(3-(4-(1,1,1-trifluoropropyl-2-yl)-4*H*-1, 2,4-triazol-3-yl)phenyl)benzamide;
2-fluoro-5-(2-(4-fluorophenyl)cyclopropyl-1-formamido)-*N*-(6-(4-isopropyl-4*H*-1,2,4-triazo 1-3-yl)pyridin-2-yl)-4-methylbenzamide;
5-(cyclobutylformamido)-2-fluoro-*N*-(6-(4-isopropyl-4*H*-1,2,4-triazol-3-yl)pyridin-2-yl)-4-methylbenzamide;
2-fluoro-*N*-(6-(4-isopropyl-4*H*-1,2,4-triazol-3-yl)pyridin-2-yl)-4-methyl-5-(1-(trifluorometh yl)cyclopropyl-1-formamido)benzamide;
2-fluoro-*N*-(6-(4-isopropyl-4*H*-1,2,4-triazol-3-yl)pyridin-2-yl)-4-methyl-5-(1-(fluoro)cyclop ropyl-1-formamido)benzamide;
2-fluoro-5-((1R,2R)-2-fluorocyclopropyl-1-formamido)-*N*-(6-(4-isopropyl-4*H*-1,2,4-triazol-3-yl)pyridin-2-yl)-4-methylbenzamide;
2-fluoro-5-((1R,2S)-2-fluorocyclopropyl-1-formamido)-*N*-(6-(4-isopropyl-4*H*-1,2,4-triazol-3-yl)pyridin-2-yl)-4-methylbenzamide;
(*S*)-5-(cyclopentylformamido)-2-fluoro-4-methyl-*N*-(6-(4-(1,1,1-trifluoropropyl-2-yl)-4*H*-1, 2,4-triazol-3-yl)pyridin-2-yl)benzamide; and
(*S*)-5-(cycloheptylformamido)-2-fluoro-4-methyl-*N*-(6-(4-(1,1,1-trifluoropropyl-2-yl)-4*H*-1, 2,4-triazol-3-yl)pyridin-2-yl)benzamide.

11. A method for preparing the compound of formula I according to claim 1, comprising performing a condensation reaction between a compound of formula II or an acyl chloride thereof and a compound of formula III under catalysis of a base, 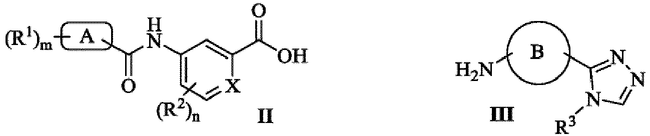 wherein each variable is as defined in claim 1.

12. A method for preparing the compound of formula I according to claim 1, comprising the following reaction steps with a compound of formula II and a compound of IV-1 as starting materials, 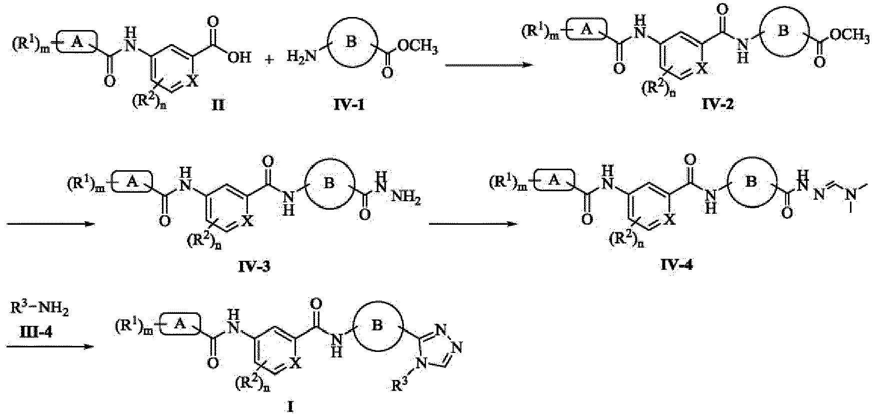 wherein each variable is as defined in claim 1.

13. A preparation method for the compound of formula I according to claim 1, comprising performing a condensation reaction between a compound of formula V and a compound of formula II-4 under the catalysis of a base, 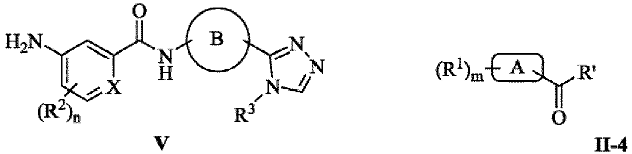 wherein, R' is OH or Cl; and the other variables are as defined in claim 1.

14. The preparation method according to claim 11 or 13, wherein the base catalystis selected from TEA, DIPEA and Py.

15. The preparation method according to claim 13, wherein the condensation reaction is carried out in the presence of a condensing agent selected from HATU, HOBt, PyBOP and T₃P.

16. The preparation method according to claim 11, wherein the compound of formula II is prepared by the following synthesis route, 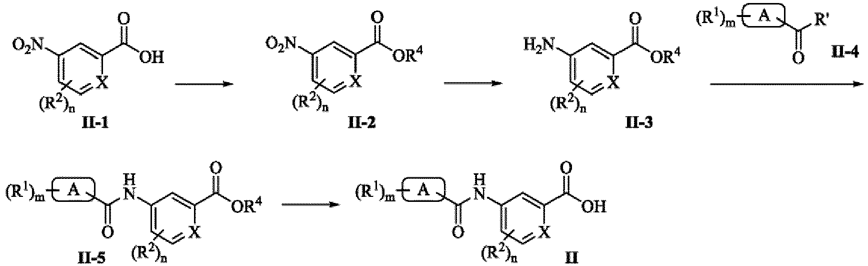 wherein, R' is OH or Cl; R⁴ is alkyl; and other variables are as defined in claim 1.

17. The preparation method according to claim 11 or 12, wherein the compound of formula III is prepared by the following synthesis route, 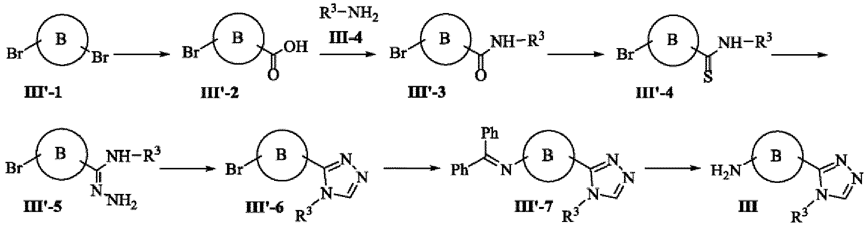 wherein each variable is as defined in claim 1.

18. A pharmaceutical composition comprising the compound of formula I according to any one of claims 1 to 10 and optionally a pharmaceutically acceptable carrier, adjuvant or diluent.

19. Use of the compound according to any one of claims 1 to 10 in the manufacture of a medicament for the treatment or prevention of diseases associated with ASK1 kinase.

20. The use according to claim 19, wherein the diseases associated with ASK1 kinase are selected from inflammatory diseases, metabolic diseases, autoimmune diseases, cardiovascular diseases, neurodegenerative diseases and cancers.

21. Use of the pharmaceutical composition according to claim 18 in the manufacture of a medicament for the treatment or prevention of a disease associated with ASK1 target.

22. The use according to claim 21, wherein the diseases associated with ASK1 target are selected from inflammatory diseases, metabolic diseases, autoimmune diseases, cardiovascular diseases, neurodegenerative diseases, cancers, and other diseases.
